(19) Europäisches Patentamt
European Patent Office
Office européen des brevets

(11) **EP 4 470 991 A1**

(12) **EUROPEAN PATENT APPLICATION**
published in accordance with Art. 153(4) EPC

(43) Date of publication:
04.12.2024 Bulletin 2024/49

(51) International Patent Classification (IPC):
$C04B\ 35/488^{(2006.01)}$    $A61C\ 13/00^{(2006.01)}$
$A61C\ 13/08^{(2006.01)}$    $C01G\ 25/02^{(2006.01)}$

(21) Application number: 23746977.0

(52) Cooperative Patent Classification (CPC):
A61C 13/00; A61C 13/08; A61K 6/818;
C01G 25/02; C04B 35/488

(22) Date of filing: 25.01.2023

(86) International application number:
PCT/JP2023/002253

(87) International publication number:
WO 2023/145766 (03.08.2023 Gazette 2023/31)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 27.01.2022 JP 2022010606

(71) Applicant: Tosoh Corporation
Yamaguchi 746-8501 (JP)

(72) Inventors:
• USHIO, Yuki
Shunan-shi Yamaguchi 746-8501 (JP)
• TSUKIMORI, Takashi
Shunan-shi Yamaguchi 746-8501 (JP)
• NAGAYAMA, Hitoshi
Shunan-shi Yamaguchi 746-8501 (JP)

(74) Representative: Müller-Boré & Partner
Patentanwälte PartG mbB
Friedenheimer Brücke 21
80639 München (DE)

(54) **POWDER AND PRODUCTION METHOD THEREFOR**

(57) Provided are a powder which contains zirconia as a main component and at least a transition metal element and from which a calcined body having mechanical properties more suitable for calcined body processing is obtained, a method for producing the powder and at least one of a calcined body, a sintered body obtained from the powder and methods for producing them. A powder of zirconia comprises a stabilizing element and a transition metal element, in which in a frequency distribution of element ratios of the transition metal element/zirconium plotted at intervals of 0.005, a difference between a minimum value and a maximum value of the transition metal element/zirconium is less than 0.25.

FIG. 9

EP 4 470 991 A1

## Description

TECHNICAL FIELD

The present disclosure relates to a powder containing zirconia as a main component and a method for producing the powder.

BACKGROUND ART

Zirconia ($ZrO_2$, zirconium dioxide) sintered bodies containing coloring components are used in a wide range of applications, such as members for portable electronic devices, decorative members, and dental prostheses. Zirconia sintered bodies have high strength and are difficult to process. For this reason, when a sintered body having a complex shape, such as a dental prosthesis, is obtained, first, a green body (green compact) containing zirconia and a coloring component is heat-treated at a temperature lower than the sintering temperature to obtain zirconia in a state having a strength suitable for processing, i.e., a so-called calcined body (semi-sintered body or pre-sintered body). Next, the calcined body is cut into a desired shape using CAD/CAM, and then the processed calcined body is sintered to produce a sintered body.

Lanthanoide rare-earth elements and transition metal elements are mainly used as coloring components for zirconia. Transition metal elements are widely used as coloring components because they are inexpensive and can easily give sintered bodies with the desired color tone.

For example, in Patent Document 1, in order to obtain a sintered body having a color suitable for a dental prosthesis, it has been studied to form a powder containing zirconia and a compound, as a coloring component, of a transition metal composed of iron or cobalt into a compact and to sinter the compact.

In Patent Document 2, in order to obtain a sintered body having a color suitable for a dental prosthesis, it has been studied to immerse a porous calcined body in a coloring solution containing iron ions and then sinter the resulting calcined body.

PRIOR ART DOCUMENTS

PATENT DOCUMENTS

Patent Document 1: U.S. Patent No. 9,428,422
Patent Document 2: European Patent Application Publication No. 3892254

DISCLOSURE OF INVENTION

TECHNICAL PROBLEM

In a method disclosed in Patent Document 2, gradual impregnation with a coloring component is performed from the surface to the inside. For this reason, the concentration of the coloring component is high on the surface of the calcined body and low in the inside, and the coloring component is contained in the calcined body with a concentration gradient from the surface to the inside. Thus, the resulting sintered body is likely to have a difference in color tone between the surface and the inside. In addition, when a calcined body that has already been processed is reprocessed, the color tone of the resulting sintered body may be visually recognized as being different from the color tone before reprocessing. In contrast, the method disclosed in Patent Document 1 makes it possible to obtain a calcined body and a sintered body in which the coloring component is contained almost uniformly from the surface to the inside of the calcined body. Therefore, the calcined body obtained from the powder of Patent Document 1 is suitable for calcined body processing. In addition, a change in color tone before and after reprocessing is small, compared with the calcined body obtained from Patent Document 2. Recent improvements in processing technology have led to finer processing. As a result, there is a demand for a calcined body having more uniform processing characteristics than the calcined body obtained from the powder described in Patent Document 1.

It is an object of the present disclosure to provide a powder which contains zirconia as a main component and at least a transition metal element, and from which a calcined body having mechanical properties more suitable for calcined body processing is obtained, a method for producing the powder and at least one of a calcined body, a sintered body obtained from the powder and methods for producing them. It is another object of the present disclosure to provide at least one of a powder of colored zirconia suitable as a raw material for a calcined body having excellent workability and a method for producing the powder, and further to provide at least one of a calcined body and a sintered body obtained from the powder.

SOLUTION TO PROBLEM

In the present disclosure, the workability of a calcined body containing zirconia as a main component and a transition metal element has been studied. As a result, we have noticed that, compared with a calcined body composed of transition metal element-free zirconia, a calcined body containing zirconia as a main component and a transition metal element not only exhibits accelerated sintering shrinkage, but also exhibits non-uniform sintering shrinkage, thereby resulting in reduced workability. Furthermore, we have focused on the relationship between the dispersibility of the transition metal element in the powder and the workability of the resulting calcined body, and have found that a calcined body with higher workability is obtained by controlling the dispersibility of the transition metal element in the powder. Moreover, we have found a powder having such dispersibility of a transition metal element, a powder of zirconia colored by the coloring of a transition metal element and suitable as a raw material for a calcined body having excellent workability and methods for producing these powders.

Specifically, the present invention is defined in the claims, and the gist of the present disclosure is described below

[1] A powder of zirconia, comprising a stabilizing element and a transition metal element, in which in a frequency distribution of element ratios of the transition metal element/zirconium plotted at intervals of 0.005, a difference between a minimum value and a maximum value of the transition metal element/zirconium is less than 0.25.

[2] The powder described in [1], in which the transition metal element is one or more selected from the group consisting of titanium (Ti), chromium (Cr), manganese (Mn), iron (Fe), cobalt (Co), nickel (Ni), copper (Cu), niobium (Nb), vanadium (V), molybdenum (Mo), technetium (Tc), ruthenium (Ru), rhodium (Rh), palladium (Pd) and silver (Ag).

[3] The powder described in [1] or [2], in which the stabilizing element is one or more selected from the group consisting of yttrium (Y), calcium (Ca), magnesium (Mg), terbium (Tb) and erbium (Er).

[4] The powder described in any one of [1] to [3], in which the powder contains one or more selected from the group consisting of alumina ($Al_2O_3$), silica ($SiO_2$) and germania ($GeO_2$).

[5] The powder described in any one of [1] to [4], in which a total frequency of the transition metal element/zirconium of 0.05 or more in the frequency distribution is 2.5% or less.

[6] The powder described in any one of [1] to [5], in which the powder has a BET specific surface area of 8 $m^2$/g or more and 15 $m^2$/g or less.

[7] The powder described in any one of [1] to [6], in which the powder has a bulk density of 1.10 g/$cm^3$ or more and 1.40 g/$cm^3$ or less.

[8] The powder described in any one of [1] to [7], in which when a disk-shaped green body obtained by filling 3.0 g of the powder into a mold having a diameter of 25 mm, performing uniaxial pressing at a pressure of 49 MPa and then performing CIP treatment at a pressure of 196 MPa is calcined under the following conditions to provide a calcined body, a shrinkage percentage determined from the following formula is less than 4.0%:

calcination temperature: 1,000°C,
calcination time: 1 hour,
rate of temperature increase: 50 °C/hour,
calcination atmosphere: air atmosphere,
rate of temperature decrease: 300 °C/hour and

$$\text{shrinkage percentage [\%]} = \{(25 - \text{diameter of calcined body) [mm]}/25\text{ [mm]}\} \times 100$$

$$\cdots (1).$$

[9] The powder described in any one of [1] to [8], in which the powder is a granulated powder.

A method for producing the powder described in any one of [1] to [8], comprising a step of drying a composition containing hydrated zirconia, a stabilizing element source, a transition metal element source and a solvent to provide a dry powder, and a step of subjecting the dry powder to heat treatment at a temperature lower than a sintering temperature to provide a calcined powder.

The method described in [10], in which the transition metal element source is at least one of an oxide and a chloride of one or more selected from the group consisting of titanium (Ti), chromium (Cr), manganese (Mn), iron (Fe), cobalt (Co), nickel (Ni), copper (Cu), niobium (Nb), molybdenum (Mo), technetium (Tc), ruthenium (Ru), rhodium (Rh), palladium (Pd) and silver (Ag).

The method described in [10] or [11], in which the heat treatment temperature in the heat treatment is 1,200°C or lower.

A green body, comprising the powder described in any one of [1] to [9].

A method for producing a calcined body, comprising a step of calcining the green body described in [13].

A method for producing a sintered body, comprising a step of sintering at least one of a green body containing the powder described in any one of [1] to [9] and a calcined body obtained by calcining the green body.

ADVANTAGEOUS EFFECTS OF INVENTION

The present disclosure can provide a powder which contains zirconia as a main component and at least a transition metal element and from which a calcined body having mechanical properties more suitable for calcined body processing is obtained, a method for producing the powder, and at least one of a calcined body and a sintered body obtained from the powder and methods for producing them. The present disclosure can also provide at least one of a powder of colored zirconia suitable as a raw material for a calcined body having excellent workability and a method for producing the powder, and further provide at least one of a calcined body and a sintered body obtained from the powder.

BRIEF DESCRIPTION OF DRAWINGS

[Fig. 1] A schematic diagram illustrating the arrangement of calcined bodies during sintering in Measurement Example 1.

[Fig. 2] An element frequency distribution (histogram) of a powder of Example 1.

[Fig. 3] An element frequency distribution (histogram) of a powder of Comparative Example 1.

[Fig. 4] An elemental mapping of iron in Example 1.

[Fig. 5] An elemental mapping of iron in Comparative Example 1.

[Fig. 6] An elemental mapping of cobalt in Example 2.

[Fig. 7] An elemental mapping of manganese in Example 3.

[Fig. 8] An elemental mapping of nickel in Example 4.

[Fig. 9] An elemental mapping of iron in Example 8.

[Fig. 10] An elemental mapping of iron in Example 9.

DESCRIPTION OF EMBODIMENTS

A powder of the present disclosure will be described with reference to an example of an embodiment. The terms used in the present embodiment are described below.

The term "composition" refers to a substance having a certain composition. Examples thereof include one or more selected from the group consisting of a powder, a green body, a calcined body and a sintered body. The term "zirconia composition" refers to a composition containing zirconia as a main component, and to a composition essentially composed of zirconia.

The term "powder" refers to a composition that is a collection of a powder particle (at least one of a primary particle and a secondary particle) and that has flowability. The term "zirconia powder" refers to a powder containing zirconia as a main component, and to a powder essentially composed of zirconia.

The term "granulated powder" refers to a composition that is a collection of the aggregate of powder particles (granulated particles) and that has flowability, and in particular, to a composition in which the powder particles have been slowly aggregated. The "zirconia granulated powder" refers to a granulated powder containing zirconia as a main component, and to a granulated powder essentially composed of zirconia.

The term "green body" refers to a composition having a certain shape composed of powder particles agglomerated by physical forces, and in particular, to a composition in which no heat treatment has been performed after imparting the shape (for example, after forming). The term "zirconia green body" refers to a formed body containing zirconia as a main component, and to a green body essentially composed of zirconia. The term "green compact" is used interchangeably with "green body".

The term "calcined body" refers to a composition having a certain shape composed of fused particles, and to a composition subjected to heat treatment at a temperature lower than a sintering temperature. The term "zirconia calcined body" refers to a calcined body containing zirconia as a main component, and to a calcined body essentially composed of zirconia.

The term "sintered body" refers to a composition having a certain shape composed of crystal grains, and to a composition subjected to heat treatment at a temperature higher than or equal to a sintering temperature. The term "zirconia sintered body" refers to a sintered body containing zirconia as a main component, and to a sintered body essentially composed of zirconia.

The term "main component" refers to a component serving as the main phase (matrix, base material, parent phase) in a composition, and to a component that preferably accounts for 75 mass% or more, 85 mass% or more, 90 mass% or more, 95 mass% or more, 98 mass% or more, or 99 mass% or more, and 100 mass% or less, or less than 100 mass%, of the composition.

The term "stabilizing element" refers to an element that stabilizes the crystal phase of zirconia by solid-solution formation with zirconia.

The term "BET specific surface area" refers to a specific surface area $[m^2/g]$ measured by a multipoint BET method (five points) using nitrogen as an adsorption gas in accordance with JIS R 1626, and in particular, to a BET specific surface area measured under the following conditions.

Adsorption medium: $N_2$
Adsorption temperature: -196°C
Pretreatment conditions: degassing treatment in air atmosphere at 250°C for 1 hour or more

The BET specific surface area can be measured with a typical specific surface area measuring device (for example, TriStar II 3020, manufactured by Shimadzu Corporation).

The term "average particle size" refers to D50 in the volume particle size distribution of a powder measured by a wet process. The average particle size can be measured with a typical device (for example, MT3300EXII, manufactured by Microtrac Bell). As a measurement sample, a slurry prepared by dispersing a powder, from which slow aggregation has been eliminated by dispersion treatment such as ultrasonic treatment, in pure water may be used. The volume particle size distribution is preferably measured by a wet process at a pH of the slurry of 3.0 to 6.0.

The term "average granule size" refers to D50 in the volume particle size distribution of a granulated powder measured by a dry process. The average granule size can be measured with a typical device (for example, MT3100II, manufactured by Microtrac Bell). As a measurement sample, a granulated powder in a slowly aggregated state may be used as it is without being subjected to dispersion treatment such as ultrasonic treatment.

The term "bulk density" refers to a density (bulk density) measured by a method according to JIS R 1628.

The term "average crystal grain size" refers to the average size of crystal grains constituting a sintered body. The average crystal grain size is determined by observing a surface of the sintered body with a scanning electron microscope (hereinafter also referred to as a "SEM") and performing image analysis of the SEM observation image obtained by observation.

The SEM observation in measuring the average crystal grain size may be performed with a typical scanning electron microscope (for example, JSM-IT500LA, manufactured by JEOL Ltd.). The SEM observation may be performed by appropriately setting the observation magnification in such a manner that the number of crystal grains to be image-analyzed (crystal grains whose crystal grain boundaries are observed continuously in the SEM observation image (described later)) is 450 ± 50. To reduce variations in crystal grains observed due to differences in the SEM observation locations, the average crystal grain size may be determined from two or more SEM observation images, or even three or more and five or less SEM observation images, such that the total number of crystal grains observed is the above-mentioned number of crystal grains. The SEM observations are required to be performed under the conditions listed below.

Acceleration voltage: 15 kV
Irradiation current: 40 nA
Observation magnification: 5,000× to 10,000x

Image analysis of the SEM observation image may be performed using image analysis software (for example, Mac-View Ver. 5, manufactured by Mountech). Specifically, crystal grains whose crystal grain boundaries are observed continuously in the SEM observation image are extracted, and the area $[\mu m^2]$ of each extracted crystal grain is calculated. The determined area is converted into the diameter $[\mu m]$ of a circle having the same area as the determined area. The resulting diameter (Heywood diameter; hereinafter, also referred to as the "equivalent circle diameter") may be regarded as the crystal grain size of each crystal grain. The average value of the equivalent circle diameters of the extracted crystal grains may be regarded as the average crystal grain size of the sintered body.

The term "powder X-ray diffraction pattern" refers to a powder X-ray diffraction (hereinafter, referred to as "XRD") pattern obtained by subjecting an XRD pattern of a composition obtained by XRD measurement under the following conditions to smoothing processing and background removal processing using an analysis program (for example, integrated powder X-ray analysis software PDXL Ver. 2.2, manufactured by Rigaku Corporation) attached to the X-ray diffractometer.

> Radiation source: Cu K$\alpha$ radiation ($\lambda$ = 0.15418 nm)
> Measurement mode: continuous scan
> Scan speed: 2 °/minute
> Measurement range: 2$\theta$ = 26° to 33° 2$\theta$ = 72° to 76°
> Accelerating voltage·current: 40 mA·40 kV
> Divergence height-limiting slit: 10 mm
> Divergence/entrance slit: 1°
> Receiving slit: open
> Detector: semiconductor detector (D/teX Ultra)
> Filter: Ni filter
> Goniometer radius: 185 mm

The XRD measurement can be performed with a typical X-ray diffractometer (for example, Ultima IV, manufactured by Rigaku Corporation). A surface of a calcined body is polished using sandpaper with a grit number of #400 in accordance with JIS R 6001-2, and then lapped using a diamond abrasive with a particle size of 3 $\mu$m to prepare a measurement sample. The lapped surface may be subjected to XRD measurement. A surface of a sintered body is polished to a surface roughness Ra $\leq$ 0.02 $\mu$m to prepare a measurement sample. The polished surface may be subjected to XRD measurement.

The term "XRD peak" refers to a peak having the peak maximum at 2$\theta$ detected in an XRD pattern obtained in the above-mentioned XRD measurement. In the present embodiment, "not have an XRD peak" indicates that the XRD peak is not detected in an XRD pattern obtained by the above-mentioned XRD measurement.

Examples of an XRD peak corresponding to a crystal plane of zirconia include XRD peaks each having a peak maximum at 2$\theta$ described below.

> XRD peak corresponding to monoclinic (111) plane: 2$\theta$ = 31 $\pm$ 0.5°
> XRD peak corresponding to monoclinic (11-1) plane: 2$\theta$ = 28 $\pm$ 0.5°
> XRD peak corresponding to tetragonal (111) plane: 2$\theta$ = 30 $\pm$ 0.5°
> XRD peak corresponding to cubic (111) plane: 2$\theta$ = 30 $\pm$ 0.5°

The XRD peak corresponding to the tetragonal (111) plane and the XRD peak corresponding to the cubic (111) plane are measured as a single overlapping peak.

The term "T+C phase ratio" refers to the ratio of the integrated intensity of the XRD peaks of tetragonal and cubic zirconia to the total integrated intensity of the XRD peaks of tetragonal, cubic and monoclinic zirconia in an XRD pattern obtained by the above-mentioned XRD measurement. The "M phase ratio" refers to the ratio of the integrated intensity of the XRD peak of monoclinic zirconia to the total integrated intensity of the XRD peaks of tetragonal, cubic and monoclinic zirconia in an XRD pattern obtained by the above-mentioned XRD measurement. These can be calculated using the following formulae.

$$f_{T+C} = [I_t(111) + I_c(111)]/[I_m(111) + I_m(11\text{-}1) + I_t(111) + I_c(111)]$$

$$f_M = 1 - f_{T+C}$$

In the above formula, $f_{T+C}$ is the T+C phase ratio, $f_M$ is the M phase ratio, $I_t(111)$ is the integrated intensity of the tetragonal (111) plane, $I_c(111)$ is the integrated intensity of the cubic (111) plane, $I_m(111)$ is the integrated intensity of the monoclinic (111) plane, $I_m(11\text{-}1)$ is the integrated intensity of the monoclinic (11-1) plane, and $I_t(111) + I_c(111)$ corresponds to the integrated intensity of the XRD peak having a peak maximum at 2$\theta$ = 30 $\pm$ 0.5°.

The integrated intensity of each XRD peak is a value obtained by analyzing an XRD pattern using an analysis program (for example, integrated powder X-ray analysis software PDXL Ver. 2.2, manufactured by RIGAKU Corporation) attached to an X-ray diffractometer.

The term "measured density" refers to the value [g/cm$^3$] calculated from the mass [g] relative to the sample volume [cm$^3$]. For the mass, the mass determined by weighing a sample may be used. For the volume, the volume determined by shape measurement may be used for a green body and a calcined body. For a sintered body, the volume obtained by Archimedes' method according to JIS R 1634 may be used. In Archimedes' method, ion-exchanged water may be used as a solvent, and pretreatment may be performed by a boiling method.

The term "total light transmittance" refers to the percentage [%] of transmitted light (the sum of linear transmitted light and diffuse transmitted light) to incident light, measured in accordance with JIS K 7361-1 for a measurement sample having a thickness of $1.0 \pm 0.1$ mm. As the measurement sample, a disk-shaped sintered body having a sample thickness of $1.0 \pm 0.1$ mm and a surface roughness Ra $\leq 0.02$ $\mu$m on both surfaces may be used. As a measuring device, a hazemeter equipped with illuminant D65 as a light source (for example, a Haze Meter NDH4000, manufactured by Nippon Denshoku Industries Co., Ltd.) may be used.

The terms "color tone (L*, a*, b*)" and "chroma C*" refer to values determined by using values measured in SCI mode with a spectrocolorimeter (for example, CM-700d, manufactured by Konica Minolta Inc.) equipped with an illumination/light-receiving optical system conforming to the geometric condition c of JIS Z 8722. A specific example of the measurement method is a method in which a zero calibration box is disposed on a measurement sample and measurement is performed under the following conditions (what is called black back measurement).

    Light source: illuminant D65
    Viewing angle: 2°
    Measurement mode: SCI

The color tone of a sintered body may be measured using a measurement sample prepared by horizontally cutting out a freely-selected portion of the sintered body and processing the cut portion to a sample thickness of $1.0 \pm 0.1$ mm.

The term "lightness L*" refers to an index indicating brightness. The lightness L* has a value of 0 or more and 100 or less. The terms "hue a*" and "hue b*" refer to indices that indicate color tone. Each of hue a* and hue b* has a value of -100 or more and 100 or less. The term "chroma C*" refers to an index indicating vividness. The chroma C* is determined from the hues a* and b* by means of $C^* = \{(a^*)^2 + (b^*)^2\}^{05}$.

The term "three-point bending strength" refers to a value measured by a method in accordance with JIS R 1601. A measurement sample used may have a pillar shape with a width of 4 mm, a thickness of 3 mm and a length of 45 mm and may be subjected to measurement at a span of 30 mm under a load applied in the horizontal direction of the measurement sample.

The term "Vickers hardness" refers to one of the indices that indicate workability. The Vickers hardness is a value measured with a typical Vickers hardness tester (for example, Q30A, manufactured by Qness) equipped with a square-pyramidal diamond indenter. In the measurement, the indenter is statically pressed onto the surface of a measurement sample, and the diagonal length of an indentation mark formed on the surface of the measurement sample is measured. The Vickers hardness may be calculated from the diagonal length using the following formula.

$$Hv = F/\{d^2/2\sin(\alpha/2)\}$$

In the above formula, Hv is the Vickers hardness (HV), F is the measurement load (1 kgf), d is the diagonal length of an indentation mark (mm), and $\alpha$ is the angle between opposite faces of the indenter (136°).

The Vickers hardness may be measured under the following conditions.

    Measurement sample: a disk shape having a thickness of $3.0 \pm 0.5$ mm
    Measurement load: 1 kgf

As pretreatment before the measurement, it is sufficient that the measurement surface of the measurement sample be polished with #800 waterproof abrasive paper to remove asperities exceeding 0.1 mm.

The term "pressureless sintering" refers to a method of sintering an object to be sintered (such as a green body or calcined body) by heating the object to a sintering temperature or higher without applying an external force to the object during sintering.

<Powder>

A powder of an embodiment is a powder of zirconia, the powder comprising a stabilizing element and a transition metal element, in which in a frequency distribution of element ratios of the transition metal element/zirconium plotted at intervals of 0.005, the difference between the minimum value and the maximum value of the transition metal element/zirconium is less than 0.25, and the powder is a powder of colored zirconia colored by the transition metal element.

The powder of the present embodiment is a powder of zirconia, the powder containing a stabilizing element and a transition metal element. The powder of the present embodiment may be considered as a powder containing zirconia, a stabilizing element and a transition metal element, the powder containing zirconia as a main component, or even as a zirconia powder containing a stabilizing element and a transition metal element. The powder of the present embodiment is preferably a powder of zirconia with which a stabilizing element forms a solid solution, the powder containing a transition metal element.

The powder of the present embodiment is a powder of zirconia, that is, a powder containing zirconia as a main component. In the powder of the present embodiment, the zirconia is preferably zirconia with which a stabilizing element forms a solid solution (hereinafter, also referred to as "stabilizing element-dissolved zirconia").

Zirconia may contain incidental impurities such as hafnia ($HfO_2$). In the calculation of the composition and a composition-based value such as density in the present embodiment, the calculation may be performed by regarding hafnia as zirconia.

The powder of the present embodiment contains a stabilizing element. Examples of the stabilizing element include rare-earth elements. Specific examples thereof include one or more selected from the group consisting of yttrium (Y), calcium (Ca), magnesium (Mg), terbium (Tb) and erbium (Er). The stabilizing element is preferably one or more selected from the group consisting of yttrium, terbium and erbium. The stabilizing element is preferably an element that does not affect the color tone of zirconia. Specifically, the stabilizing element is preferably one or more selected from the group consisting of yttrium, calcium and magnesium, more preferably yttrium.

Preferably, at least a part of the stabilizing element forms a solid solution with zirconia. More preferably, the stabilizing element forms a solid solution with zirconia (in other words, the powder of the present embodiment does not contain a stabilizing element that does not form a solid solution). However, as long as the effect of the powder of the present embodiment is provided, the powder of the present embodiment may contain a stabilizing element that does not form a solid solution with zirconia.

In the present embodiment, the absence of a stabilizing element that does not form a solid solution can be verified by the absence of an XRD peak derived from a compound of the stabilizing element in an XRD pattern.

The amount of the stabilizing element in the powder of the present embodiment (hereinafter, also referred to as the "amount of stabilizing element", and when the stabilizing element is yttrium or the like, it is also referred to as the "amount of yttrium" or the like) may be an amount that partially stabilizes the zirconia crystal phase, and may be an amount such that zirconia has a crystal phase in which a tetragonal phase and a cubic phase are main phases. The amount of the stabilizing element is, for example, 2 mol% or more or 2.5 mol% or more and 15 mol% or less or 7.5 mol% or less, and preferably 2 mol% or more and 15 mol% or less, 2.5 mol% or more and 15 mol% or less or 3.5 mol% or more and 5.9 mol% or less. When the stabilizing element is yttrium, the amount of the stabilizing element (amount of yttrium) may be 3 mol% or more, 3.3 mol% or more, 3.5 mol% or more or 3.6 mol% or more, and 6.5 mol% or less, 6 mol% or less, 5.5 mol% or less or 5.2 mol% or less. The amount of the stabilizing element is preferably 2 mol% or more and 6.5 mol% or less, 3 mol% or more and 6.5 mol% or less, 3.3 mol% or more and 6 mol% or less, 3.5 mol% or more and 5.5 mol% or less, 3.6 mol% or more and 5.1 mol% or less or 4.8 mol% or more and 5.5 mol% or less, because the transition metal element is more likely to be uniformly dispersed.

The amount of the stabilizing element may be determined as the proportion [mol%] of the stabilizing element [mol] on an oxide basis in the total [mol] of zirconia and the stabilizing element on an oxide basis. The oxide equivalents of the respective stabilizing elements are $Y_2O_3$ for yttrium, $CaO$ for calcium, $MgO$ for magnesium, $Tb_4O_7$ for terbium and $Er_2O_3$ for erbium.

The powder of the present embodiment contains a transition metal element. The transition metal element in the powder of the present embodiment particularly indicates a transition metal element contained in the powder as a coloring component. As a result, the sintered body obtained from the powder of the present embodiment exhibits the desired color tone derived from the transition metal element, in particular a color tone that is difficult to exhibit with lanthanoide rare-earth elements and the like.

The transition metal element contained in the powder of the present embodiment is a transition metal element that can color zirconia, preferably a transition metal element other than zirconium and hafnium, more preferably at least one of a 3d transition metal element (3d transition element) and a 4d transition metal element (4d transition element) other than zirconium, still more preferably a 3d transition metal element and still further more preferably a 3d transition metal element other than vanadium. For the sake of convenience, in the present embodiment, the transition metal element does not include lanthanoid rare-earth elements, and refers to a transition metal element other than lanthanoid rare-earth elements. Specific examples of the transition metal element include one or more selected from the group consisting of titanium (Ti), chromium (Cr), manganese (Mn), iron (Fe), cobalt (Co), nickel (Ni), copper (Cu), niobium (Nb), vanadium (V), molybdenum (Mo), technetium (Tc), ruthenium (Ru), rhodium (Rh), palladium (Pd) and silver (Ag). The transition metal element is preferably one or more selected from the group consisting of titanium, chromium, manganese, iron, cobalt, nickel and copper, more preferably one or more selected from the group consisting of titanium, manganese, iron and cobalt, still more preferably one or more selected from the group consisting of titanium, iron and manganese, still further more preferably at least one of iron and titanium and particularly preferably iron.

For example, in the case of obtaining a sintered body exhibiting a yellow-based color, the transition metal element is, for example, one or more selected from the group consisting of chromium, iron and vanadium. Iron is preferred. In the case of obtaining a sintered body exhibiting a green-based color, the transition metal element is, for example, nickel. In the case of obtaining a sintered body exhibiting a gray-based color, the transition metal element is, for example, manganese. In the case of obtaining a sintered body exhibiting a blue-based color, the transition metal element is, for example, cobalt. In order to obtain a sintered body exhibiting a color tone suitable for a dental prosthesis, the transition metal element contained in the powder of the present embodiment may be one or more selected from the group consisting of nickel, cobalt, manganese and iron, may be nickel, cobalt, manganese or iron, may be one or more selected from the group consisting of cobalt, manganese and iron, may be cobalt, manganese or iron, may be at least one of cobalt and iron, or may be cobalt or iron. Iron is preferred.

When the powder of the present embodiment contains vanadium (V), because the crystal phase of zirconia is likely to be transformed in an air atmosphere, the vanadium content is required to be 0 mass% or more and 0.005 mass% or less, or more than 0 mass% and 0.005 mass% or less. Preferably, the powder does not contain vanadium (not more than the measurement limit).

The transition metal element content of the powder of the present embodiment (hereinafter also referred to as the "amount of transition metal", and when the transition metal element is iron or the like, it is also referred to as the "amount of iron" or the like) is required to be an amount such that the crystal particles of the transition metal compound in the sintered body obtained from the powder of the present embodiment are not precipitated. For example, the content is more than 0 mass%, 0.01 mass% or more, 0.04 mass% or more or 0.1 mass% or more, and 3 mass% or less, 2 mass% or less, less than 2 mass%, 1 mass% or less or 0.5 mass% or less. Preferably, the amount of the transition metal is more than 0 mass% and 3 mass% or less, 0.01 mass% or more and 3 mass% or less, 0.04 mass% or more and 2 mass% or less, 0.04 mass% or more and less than 2 mass%, 0.04 mass% or more and 1 mass% or less or 0.1 mass% or more and 2 mass% or less.

For example, the amount of iron in the powder of the present embodiment is 0.01 mass% or more or 0.1 mass% or more, and 3 mass% or less, 2 mass% or less, 1 mass% or less or 0.5 mass% or less. Preferably, the amount of iron is 0.01 mass% or more and 3 mass% or less, 0.1 mass% or more and 3 mass% or less, 0.1 mass% or more and 2 mass% or less or 0.1 mass% or more and 0.5 mass% or less. For example, the amount of cobalt, the amount of nickel, and the amount of manganese in the powder of the present embodiment are each 0.01 mass% or more or 0.03 mass% or more, and 1 mass% or less, 0.5 mass% or less or 0.1 mass% or less, and are each preferably 0.01 mass% or more and 0.5 mass% or less, or 0.03 mass% or more and 0.1 mass% or less.

The amount of the transition metal may be calculated as the proportion by mass [mass%] of the transition metal element [g] on an oxide basis to the total mass [g] of zirconia, the stabilizing element on an oxide basis and the metal element on an oxide basis. The oxide equivalents of transition metal elements are $TiO_2$ for titanium, $Cr_2O_3$ for chromium, $Mn_3O_4$ for manganese, $Fe_2O_3$ for iron, $Co_3O_4$ for cobalt, $NiO$ for nickel, $CuO$ for copper, $Nb_2O_3$ for niobium, $V_2O_5$ for vanadium, $Mo_2O_3$ for molybdenum, $Tc_2O_3$ for technetium, $RuO_2$ for ruthenium, $RhO_2$ for rhodium, $Pd_2O_3$ for palladium and $Ag_2O$ for silver.

In the powder of the present embodiment, in a frequency distribution of element ratios (hereinafter simply referred to as an "element frequency distribution") in which the transition metal element/zirconium is plotted at intervals of 0.005, the difference between the minimum value and the maximum value of the transition metal element/zirconium is less than 0.25. Preferably, the difference is 0.2 or less, 0.16 or less, 0.12 or less, 0.11 or less or 0.1 or less. Since the powder of the present embodiment contains zirconia as the main component, zirconia is in a substantially uniformly dispersed state. The amount of the transition metal element required for coloring zirconia, particularly for coloring zirconia used as a dental prosthetic material, is extremely small relative to the amount of zirconia. Although a small amount of transition metal element is prone to aggregation, it is considered that the transition metal element is contained in a uniformly dispersed state as compared with conventional zirconia powders containing a transition metal element as a coloring component, when the difference between the minimum value and the maximum value of M/Zr (hereinafter referred to as an "M/Zr range", or referred to as an "Fe/Zr range" or the like when the transition metal element is iron or the like) satisfies the above-mentioned value. Thus, it is considered that the aggregation of the transition metal element in the case of heat-treating the powder of the present embodiment is inhibited as compared with that of the conventional powder. As a result, a calcined body having a hardness suitable for processing (particularly CAD/CAM processing for dental prosthetic materials) is obtained. In addition, it is believed that a localized hard region is less likely to form in the resulting calcined body, resulting in a calcined body that exhibits more uniform workability. A smaller M/Zr range results in higher uniformity of the transition metal element. In the powder of the present embodiment, the transition metal element has a certain range of distribution (that is, the minimum value and the maximum value of M/Zr are different). Thus, for example, the M/Zr range is more than 0, 0.03 or more or 0.06 or more. For example, the M/Zr range is preferably more than 0 and less than 0.25, more than 0 and 0.2 or less, 0.03 or more and 0.16 or less, 0.03 or more and 0.12 or less, 0.03 or more and 0.1 or less or 0.06 or more and 0.1 or less.

To avoid the segregation of the transition metal element, excessive aggregation of the transition metal element is preferably minimized in the powder of the present embodiment. The maximum value of M/Zr in the element frequency distribution is 0.3 or less, 0.2 or less, 0.1 or less or 0.08 or less. Since the detection sensitivity for each element in EPMA measurement described later differs, the maximum value of M/Zr may differ in accordance with the type of transition metal element. When the transition metal element is iron, the maximum value of M/Zr (Fe/Zr) is, for example, 0.03 or more and 0.2 or less, 0.05 or more and 0.12 or less or 0.05 or more and 0.1 or less. Similarly, when the transition metal element is cobalt, the maximum value of M/Zr (Co/Zr) is, for example, 0.03 or more and 0.2 or less or 0.1 or more and 0.15 or less. When the transition metal element is manganese, the maximum value of M/Zr (Mn/Zr) is, for example, 0.03 or more and 0.2 or less or 0.05 or more and 0.12 or less. When the transition metal element is nickel, the maximum value of M/Zr (Ni/Zr) is, for example, 0.03 or more and 0.2 or less or 0.12 or more and 0.18 or less.

Similarly, when the transition metal element is iron, in the element frequency distribution, the total frequency where M/Zr is 0.05 or more (hereinafter also referred to as "high metal frequency") is 2.5% or less, and is preferably 2% or less, 1.5% or less, 1.0% or less, 0.5% or less, 0.1% or less or 0.05% or less. The high metal frequency may be 0% or more, more than 0% or 0.02% or more. In the case where the transition metal element is iron, when the high metal frequency is within this range, the calcined body obtained from the powder of the present embodiment is not too hard and has a hardness more suitable for processing. When the transition metal element is iron, for example, the high metal frequency is preferably 0% or more and 2.5% or less, 0% or more and 1.5% or less, 0% or more and 0.1% or less, more than 0% and 0.1% or less or more than 0% and 0.05% or less.

In order to allow the obtained calcined body to have more uniform mechanical properties, when the transition metal element is iron, the total frequency where M/Zr is less than 0.005 (hereinafter also referred to as "low metal frequency") in the element frequency distribution of the powder of the present embodiment is, for example, 6.5% or less or 5% or less, and 0% or more, more than 0% or 3% or more, preferably 0% or more and 6.5% or less, 0% or more and 5% or less or more than 0% and 5% or less.

The element frequency distribution in the present embodiment is a distribution obtained from the EPMA spectrum of the powder, and is obtained from elemental mapping of the transition metal element and zirconium by EPMA measurement.

Specifically, the element frequency distribution can be obtained by the following method. First, the SEM observation image of the powder of the present embodiment is divided into 50,000 to 66,000 regions, and each of the obtained regions is set as a measurement point. Characteristic X-rays of zirconium and the transition metal element are measured at each measurement point to obtain elemental mapping. Next, the ratio (M/Zr) of the intensity of the characteristic X-rays of the transition metal element (M) to the intensity of the characteristic X-rays of zirconium (Zr) at each measurement point is determined. From the obtained M/Zr, a histogram is created in which the class is M/Zr, the class width (the maximum value and the minimum value of M/Zr in each class) is 0.005, and the frequency value (frequency) is the number of measurement points corresponding to each class, and the histogram is used as the element frequency distribution. Specifically, M/Zr is divided into classes, with class widths of 0.005 increments, from 0 to the maximum value of M/Zr, such as 0 or more and less than 0.005, 0.005 or more and less than 0.010, ..., and the frequency of each class is plotted to obtain a histogram, which can be used as the element frequency distribution.

The conditions for SEM observation and EPMA in measuring the element frequency distribution are described below.

Acceleration voltage: 15 kV
Illumination current: 50 nA
Probe diameter: 1 μm
Acquisition time: 50 msec
Magnification: 5,000×

For SEM observation and EPMA measurement, a scanning electron microscope equipped with an electron beam microanalyzer (for example, EPMA1610, manufactured by Shimadzu Corporation, or JXA-iHP200F, manufactured by JEOL Ltd.) can be used. A resin-embedded powder (powder particles) is used as a measurement sample, and a cut surface obtained by cutting it may be subjected to SEM observation and EPMA measurement. In order to obtain accurate elemental mapping, the powder (powder particles) used as a measurement sample is preferably a granulated powder (granulated particles).

The M/Zr range is the absolute value of the difference between the maximum value of M/Zr and the minimum value of M/Zr in the element frequency distribution. The maximum value of M/Zr is the smallest value in the M/Zr class next to the class in which M/Zr is maximum in the element frequency distribution. For example, when M/Zr of the maximum class is 0.100 or more and less than 0.105, the minimum value of the next class (0.105 or more and less than 0.110), 0.105, corresponds to the maximum value of M/Zr. The minimum value of M/Zr is the minimum value of M/Zr in the smallest class in the element frequency distribution. For example, when M/Zr in the smallest class is 0 or more and less than 0.005, 0 corresponds to the minimum value of M/Zr, and when M/Zr in the smallest class is 0.005 or more and less than 0.010, 0.005 corresponds to the minimum value of M/Zr.

The high metal frequency is the proportion (%) of the total frequency in the class where M/Zr is 0.05 or more (the number of measurement points where M/Zr is 0.05 or more) in the total frequency (total number of measurement points) in the element frequency distribution. The low metal frequency is the proportion (%) of the total frequency in the class where M/Zr is less than 0.005 (the number of measurement points where M/Zr is less than 0.005) in the total frequency (total number of measurement points) in the element frequency distribution. Thus, the high metal frequency can be regarded as the proportion of the number of measurement points where the intensity of the characteristic X-rays of the transition metal element relative to the characteristic X-rays of zirconium is 0.05 or more in the total number of measurement points in elemental mapping by EPMA measurement. The low metal frequency can be regarded as the proportion of the number of measurement points where the intensity of the characteristic X-rays of the transition metal element relative to the characteristic X-rays of zirconium is less than 0.005 in the total number of measurement points in element mapping by EPMA measurement.

To adjust the sinterability, the powder of the present embodiment may contain one or more selected from the group consisting of alumina ($Al_2O_3$), silica ($SiO_2$) and germania ($GeO_2$) (hereinafter also referred to as an "additive component"), and may further contain alumina. When the additive component is contained, the temperature at the time of sintering can be lowered. The additive component content (hereinafter also referred to as the "amount of additive component", and when the additive component is alumina or the like, it is also referred to as the "amount of alumina" or the like) may be appropriately adjusted in accordance with the desired sinterability, and is, for example, 0 mass% or more, more than 0 mass%, 0.005 mass% or more, 0.01 mass% or more or 0.03 mass% or more, and less than 0.2 mass%, less than 0.15 mass%, less than 0.1 mass% or 0.08 mass% or less. For example, the amount of the additive component is preferably 0 mass% or more and less than 0.2 mass%, 0 mass% or more and 0.08 mass% or less, 0 mass% or more and 0.04 mass% or less, 0 mass% or more and 0.08 mass% or less, more than 0 mass% and less than 0.2 mass% or 0.005 mass% or more and 0.08 mass% or less.

The amount of the additive component may be determined as the proportion [mass%] of the additive component [g] on an oxide basis in the total [g] of zirconia, the stabilizing element on an oxide basis, and the metal element on an oxide basis.

The powder of the present embodiment may contain a binder. When the binder is contained, the shape retention of the green body (green compact) obtained by forming the powder of the present embodiment is further improved. The binder may be any known binder used in forming ceramics, and is preferably an organic binder. The organic binder is at least one selected from the group consisting of poly(vinyl alcohol), poly(vinyl butyrate), wax and an acrylic resin, preferably at least one of poly(vinyl alcohol) and an acrylic resin, and more preferably an acrylic resin. In the present embodiment, the acrylic resin is a polymer containing at least one of an acrylate and a methacrylate. Specifically, the acrylic resin is, for example, one or more selected from the group consisting of poly(acrylic acid), poly(methacrylic acid), an acrylic acid copolymer and a methacrylic acid copolymer and a derivative thereof. Specific examples of the acrylic resin binder include acrylic resins used for ceramic powders, and one or more selected from the group consisting of AS-1100, AS-1800 and AS-2000 (all product names, manufactured by Toagosei Co., Ltd.).

Any binder content of the powder may be used as long as the powder exhibits desired shape retention. The proportion by mass of the binder based on the mass of the powder is, for example, 0.5 mass% or more or 1 mass% or more, and 10 mass% or less or 5 mass% or less, and is preferably 0.5 mass% or more and 10 mass% or less, or 1 mass% or more and 4 mass% or less.

For example, when the powder of the present embodiment is a zirconia powder containing yttrium and erbium as stabilizing elements, iron and cobalt as transition metal elements and alumina as an additive component, the composition can be determined as given below.

$$\text{Amount of stabilizing element [mol\%]} = \{(Y_2O_3 + Er_2O_3)/(Y_2O_3 + Er_2O_3 + ZrO_2)\} \times 100$$

$$\text{Amount of yttrium [mol\%]} = \{Y_2O_3/(Y_2O_3 + Er_2O_3 + ZrO_2)\} \times 100$$

$$\text{Amount of erbium [mol\%]} = \{Er_2O_3/(Y_2O_3 + Er_2O_3 + ZrO_2)\} \times 100$$

$$\text{Amount of transition metal [mass\%]} = \{(Fe_2O_3 + Co_3O_4)/(Y_2O_3 + Er_2O_3 + ZrO_2 + Al_2O_3 + Fe_2O_3 + Co_3O_4)\} \times 100$$

$$\text{Amount of iron [mass\%]} = \{Fe_2O_3/(Y_2O_3 + Er_2O_3 + ZrO_2 + Al_2O_3 + Fe_2O_3 + Co_3O_4)\} \times 100$$

$$\text{Amount of cobalt [mass\%]} = \{Co_3O_4/(Y_2O_3 + Er_2O_3 + ZrO_2 + Al_2O_3 + Fe_2O_3 + Co_3O_4)\} \times 100$$

Amount of additive component (amount of alumina) = $\{Al_2O_3/(Y_2O_3 + Er_2O_3 + ZrO_2 + Al_2O_3 + Fe_2O_3 + Co_3O_4)\} \times 100$

The binder content [mass%] is calculated by [{(mass of powder before heat treatment) - (mass of powder after heat treatment)}/ (mass of powder before heat treatment)] $\times$ 100 from the mass of the powder of the present embodiment before and after heat treatment in air at 250°C or higher and 400°C or lower. The amount of the stabilizing element, the amount of the transition metal and the amount of the additive component in the powder containing the binder may also be determined by the above-mentioned method.

The powder of the present embodiment has XRD peaks of zirconia in its XRD pattern. The powder of the present embodiment preferably has no XRD peaks other than zirconia in its XRD pattern, i.e., the XRD pattern preferably has only XRD peaks of zirconia. The powder of the present embodiment more preferably does not have an XRD peak of a compound of the transition metal element in its XRD pattern, and still more preferably does not have an XRD peak of a compound of the stabilizing element or a compound of the transition metal element.

The XRD peaks of zirconia in the XRD pattern of the powder of the present embodiment may be XRD peaks of at least any of tetragonal, cubic and monoclinic zirconia, and preferably have at least XRD peaks of tetragonal and cubic zirconia, and mainly include XRD peaks of tetragonal and cubic zirconia.

The T+C phase ratio of the powder of the present embodiment may be 50% or more (0.5 or more), 55% or more, 60% or more, 90% or more or 92% or more, and may be 99% or less (0.99 or less) or 95% or less. The T+C phase ratio is preferably 50% or more and 99% or less, 90% or more and 99% or less, or 92% or more and 99% or less.

The proportion of the monoclinic phase in the zirconia crystal phase of the powder of the present embodiment (hereinafter also referred to as the "M phase ratio") is a value such that the sum of the T+C phase ratio and the M phase ratio is 1 (100%), and may be more than 1% or more than 5%, and may be less than 50%, less than 45%, less than 40% or less than 10%.

The BET specific surface area of the powder of the present embodiment may be 8 $m^2$/g or more, 9 $m^2$/g or more, 9.5 $m^2$/g or more or 10 $m^2$/g or more, and may be 15 $m^2$/g or less, 14 $m^2$/g or less or 13 $m^2$/g or less. The BET specific surface area is preferably 8 $m^2$/g or more and 15 $m^2$/g or less, 9 $m^2$/g or more and 14 $m^2$/g or less, 10 $m^2$/g or more and 13 $m^2$/g or less, or 10 $m^2$/g or more and 12 $m^2$/g or less because a calcined body having a hardness suitable for CAD-CAM processing for producing a dental prosthetic material can be easily obtained.

The average particle size of the powder of the present embodiment may be 0.35 $\mu$m or more or 0.4 $\mu$m or more, and 0.55 $\mu$m or less or 0.5 $\mu$m or less. A preferred average particle size is, for example, 0.35 $\mu$m or more and 0.55 $\mu$m or less, or 0.4 $\mu$m or more and 0.5 $\mu$m or less.

The powder of the present embodiment may have a bulk density of 1.10 g/$cm^3$ or more or 1.15 g/$cm^3$ or more, and 1.40 g/$cm^3$ or less or 1.35 g/$cm^3$ or less. The bulk density is preferably 1.10 g/$cm^3$ or more and 1.40 g/$cm^3$ or less, 1.20 g/$cm^3$ or more and 1.30 g/$cm^3$ or less or 1.25 g/$cm^3$ or more and 1.30 g/$cm^3$ or less.

The powder of the present embodiment may be a powder containing at least one of powder particles and granulated particles, and may be a powder containing granulated particles as a main component. The primary particles and secondary particles constituting the powder particles may have any shape, and examples thereof include one or more selected from the group consisting of an indefinite shape, a substantially spherical shape and a substantially polyhedral shape.

The powder of the present embodiment is preferably a granulated powder. In the case of the granulated powder, the formability and handleability are further improved. The granulated powder has an average granule size of, for example, 30 $\mu$m or more and 80 $\mu$m or less, even 40 $\mu$m or more and 50 $\mu$m or less.

Compared to conventional zirconia powders containing transition metal elements, the powder of the present embodiment preferably suppresses shrinkage during calcination, i.e., during heat treatment below the sintering temperature, despite containing a transition metal element. First, 3.0 g of the powder is filled into a mold having a diameter of 25 mm, subjected to uniaxial pressing at a pressure of 49 MPa and then CIP treatment at a pressure of 196 MPa to provide a disk-shaped green body. Then the green body is calcined under the following conditions to provide a calcined body. In this case, the shrinkage percentage calculated from the following formula is preferably less than 4.0%, more preferably 3.9% or less and still more preferably 3.7% or less.

Calcination temperature: 1,000°C
Calcination time: 1 hour
Rate of temperature increase: 50 °C/hour
Calcination atmosphere: air atmosphere
Rate of temperature decrease: 300 °C/hour

Shrinkage percentage [%] = {(25 - diameter of calcined body) [mm]/25 [mm]} × 100          (1)

The calcined body thus obtained has a disk shape with a diameter of 25 mm or less and a thickness of 2 ± 0.5 mm. In formula (1), the diameter of the calcined body can be measured by a known method. The average value of values obtained by measuring the length of the disk in the diametrical direction at four points using a vernier caliper may be used.

The powder of the present embodiment has a shrinkage percentage of 3.0% or more, 3.3% or more or 3.5% or more. For example, the shrinkage percentage is preferably 3.0% or more and less than 4.0%, 3.3% or more and 3.9% or less or 3.5% or more and 3.9% or less.

The powder of the present embodiment can be used for known applications of zirconia powder. For example, the powder can be used as a precursor of a sintered body used for one or more applications selected from the group consisting of a structural material, an optical material, a decorative material, a dental material, a communication material and a biomaterial. Moreover, the powder is suitable as a precursor of a dental material, a precursor of a dental prosthetic material and a precursor for at least one of dental prosthetic materials such as a crown and a bridge.

<Method for Producing Powder>

Any method for producing a powder may be used as long as the powder of the present embodiment has the above-mentioned configuration. A preferred example of a method for producing the powder of the present embodiment is a method for producing a powder, the method including the steps of drying a composition containing hydrated zirconia, a stabilizing element source, a transition metal element source and a solvent to provide a dry powder, and subjecting the dry powder to heat treatment at a temperature lower than the sintering temperature to provide a calcined powder.

The production method of the present embodiment includes a step of drying a composition containing hydrated zirconia, a stabilizing element source, a transition metal element source, and a solvent to provide a dry powder (hereinafter, also referred to as a "drying step"). This removes the water of hydration of the hydrated zirconia ($ZrO_2 \cdot nH_2O$, where n is an integer) and the solvent from the composition, thereby providing a dry powder (a powder of zirconia, containing the stabilizing element source and the transition metal element source, i.e., a powder containing zirconia as a main component and containing the stabilizing element source and the transition metal element source).

In the drying step, the hydrated zirconia is allowed to coexist with the stabilizing element source and the transition metal element source, and then dried. Thus, unlike a step of forming hydrated zirconia by mixing a precursor of hydrated zirconia and a transition metal element source, the formation of a poorly soluble transition metal element compound associated with the formation of hydrated zirconia is significantly inhibited. Thus, it is considered that even when the transition metal element source is contained in an amount of 0.05 mass% or more, even 0.1 mass% or more, aggregation of a poorly soluble transition metal compound is less likely to occur, and as a result, a calcined body having uniform workability is obtained. It is considered that the dry powder obtained through the drying step has very little localization of the stabilizing element and the transition metal element. In addition, the transition metal element can be allowed to coexist with zirconia prior to the formation of the solid solution of the stabilizing element with zirconia by heat treatment. Thus, it is considered that the aggregation of the transition metal element in the dry powder is significantly inhibited as compared with a dry powder obtained by mixing the transition metal element source with a dry powder composed of the stabilizing element source and zirconia.

In the drying step, a composition containing hydrated zirconia, a stabilizing element source, a transition metal element source, and a solvent (hereinafter, also referred to as a "raw material composition") is provided. In the hydrated zirconia, the stabilizing element source, and the transition metal element source contained in the raw material composition, the amounts of zirconia, the stabilizing element and the transition metal element may be the same as those in the composition of the above-described powder.

The stabilizing element source content of the raw material composition is, for example, 2 mol% or more or 2.5 mol% or more and 15 mol% or less or 7.5 mol% or less, and is preferably 2 mol% or more and 15 mol% or less, 2.5 mol% or more and 15 mol% or less or 3.5 mol% or more and 5.9 mol% or less. For example, when the stabilizing element source is a yttrium source, the amount of the stabilizing element source (amount of yttrium source) may be 3 mol% or more, 3.3 mol% or more, 3.5 mol% or more or 3.6 mol% or more, and 6.5 mol% or less, 6 mol% or less, 5.5 mol% or less or 5.2 mol% or less. For example, the amount of the stabilizing element source is preferably 2 mol% or more and 6.5 mol% or less, 3 mol% or more and 6.5 mol% or less, 3.3 mol% or more and 6 mol% or less, 3.5 mol% or more and 5.5 mol% or less, 3.6 mol% or more and 5.1 mol% or less or 4.8 mol% or more and 5.5 mol% or less.

The transition metal element source content of the raw material composition is, for example, more than 0 mass%, 0.01 mass% or more, 0.04 mass% or more or 0.1 mass% or more, and 3 mass% or less, 2 mass% or less, less than 2 mass%, 1 mass% or less or 0.5 mass% or less. For example, the amount of the transition metal is preferably more than 0 mass% and 3 mass% or less, 0.01 mass% or more and 3 mass% or less, 0.04 mass% or more and 2 mass% or less, 0.04 mass% or more and less than 2 mass% or 0.1 mass% or more and 2 mass% or less.

In order to disperse hydrated zirconia more easily, the pH of the raw material composition is preferably 7 or less, more preferably 1 or more or 3 or more, and 7 or less or 5 or less.

The hydrated zirconia is preferably a hydrated zirconia obtained by one or more selected from the group consisting of hydrolysis, coprecipitation and neutralization of a zirconium salt, more preferably a hydrated zirconia obtained by hydrolysis, and still more preferably a hydrated zirconia in a state obtained by hydrolysis. The zirconium salt to be subjected to hydrolysis or the like is, for example, one or more selected from the group consisting of zirconium oxychloride, zirconyl nitrate, zirconium chloride and zirconium sulfate, and zirconium oxychloride is preferred.

The hydrated zirconia is preferably contained in the raw material composition as a hydrated zirconia sol.

The stabilizing element source (hereinafter, when the stabilizing element is yttrium or the like, it is also referred to as an "yttrium source" or the like) may be at least any one of a salt and a compound containing the stabilizing element. The stabilizing element source may be one or more selected from the group consisting of an oxide, a hydroxide, an oxyhydroxide, a halide, a carbonate, a sulfate, a nitrate and an acetate containing the above-mentioned stabilizing element. The stabilizing element source is preferably one or more selected from the group consisting of an oxide, a hydroxide, an oxyhydroxide and a chloride containing a stabilizing element, more preferably at least one of a hydroxide and a chloride containing the stabilizing element. Furthermore, the stabilizing element source may be a solution containing at least one of the salt and the compound of the stabilizing element described above. The solvent in the solution may be at least one of alcohol and water, and may be water.

For example, the yttrium source is one or more selected from the group consisting of yttrium oxide, yttrium hydroxide, yttrium oxyhydroxide, yttrium chloride, yttrium carbonate, yttrium sulfate, yttrium nitrate and yttrium acetate; one or more selected from the group consisting of yttrium oxide, yttrium hydroxide, yttrium oxyhydroxide and yttrium chloride; or at least one of yttrium oxide and yttrium chloride.

For example, the erbium source is one or more selected from the group consisting of erbium oxide, erbium hydroxide, erbium oxyhydroxide, erbium chloride, erbium carbonate, erbium sulfate, erbium nitrate and erbium acetate; one or more selected from the group consisting of erbium oxide, erbium hydroxide, erbium oxyhydroxide and erbium chloride; or at least one of erbium oxide and erbium chloride.

For example, the terbium source is one or more selected from the group consisting of terbium oxide, terbium hydroxide, terbium oxyhydroxide, terbium chloride, terbium carbonate, terbium sulfate, terbium nitrate and terbium acetate; one or more selected from the group consisting of terbium oxide, terbium hydroxide, terbium oxyhydroxide and terbium chloride; or at least one of terbium oxide and terbium chloride.

For example, the calcium source is one or more selected from the group consisting of calcium oxide, calcium hydroxide, calcium oxyhydroxide, calcium chloride, calcium carbonate, calcium sulfate, calcium nitrate and calcium acetate; one or more selected from the group consisting of calcium oxide, calcium hydroxide, calcium oxyhydroxide and calcium chloride; or at least one of calcium oxide and calcium chloride.

For example, the magnesium source is one or more selected from the group consisting of magnesium oxide, magnesium hydroxide, magnesium oxyhydroxide, magnesium chloride, magnesium carbonate, magnesium sulfate, magnesium nitrate and magnesium acetate; one or more selected from the group consisting of magnesium oxide, magnesium hydroxide, magnesium oxyhydroxide and magnesium chloride; or at least one of magnesium oxide and magnesium chloride.

The raw material composition contains a transition metal element source (hereinafter, when the transition metal element is iron or the like, it is also referred to as an "iron source" or the like). The transition metal element contained in the transition metal element source may be the transition metal element described above. This inhibits the formation of coarse aggregates of the transition metal element as compared to when the transition metal element source is mixed with a powder of zirconia or a powder of zirconia containing a stabilizing element.

The transition metal element source may be at least one of a salt and a compound of a transition metal element soluble in a solvent of the raw material composition. For example, the transition metal element source is one or more selected from the group consisting of an oxide, a hydroxide, an oxyhydroxide, a halide, a carbonate, a sulfate, a nitrate and an acetate of the above-mentioned transition metal element. The transition metal element source is preferably one or more selected from the group consisting of a hydroxide, an oxyhydroxide, a chloride and an acetate of the above-mentioned transition metal element, and is more preferably at least one of an oxide and a chloride of the above-mentioned transition metal element. Furthermore, the transition metal element source may be a solution containing at least one of the salt and the compound of the transition metal element described above. The solvent of the solution may be at least one of alcohol and water, and may be water. The transition metal element source may be a salt or compound containing at least one of a 3d transition metal element and a 4d transition metal element other than zirconium. For example, the transition metal element source is one or more selected from a titanium source, a chromium source, a manganese source, an iron source, a cobalt source, a nickel source and a copper source. For example, the transition metal element is a titanium source, a chromium source, a manganese source, an iron source, a cobalt source, a nickel source or a copper source. For example, the transition metal source is preferably one or more selected from the group consisting of a manganese source, an iron source, a cobalt source and a nickel source; a manganese source, an iron source, a cobalt source or a nickel source; at least one of a cobalt source and an iron source; a cobalt source and an iron source; or an iron source.

For example, the titanium source is one or more selected from the group consisting of titanium oxide ($TiO_2$), titanium hydroxide ($Ti(OH)_2$), titanium oxyhydroxide ($TiOOH$), titanium(II) chloride ($TiCl_2$), titanium(III) chloride ($TiCl_3$), titanium(IV) chloride ($TiCl_4$), titanium sulfate ($TiSO_4$), titanium nitrate ($Ti(NO_3)_2$) and titanium acetate ($Ti(CH_3COO)_4$).

For example, the chromium source is one or more selected from the group consisting of chromium(II) oxide ($CrO$), chromium(III) oxide ($Cr_2O_3$), chromium dioxide ($CrO_2$), trichromium tetroxide ($Cr_3O_4$), chromium oxyhydroxide ($CrOOH$), chromium(II) chloride ($CrCl_2$), chromium(III) chloride ($CrCl_3$), chromium carbonate ($Cr_2(CO_3)_3$), chromium sulfate ($CrSO_4$), chromium nitrate ($Cr(NO_3)_2$) and chromium acetate ($Cr(CH_3COO)_2$); or at least one of chromium hydroxide and chromium acetate.

For example, the manganese source is one or more selected from the group consisting of manganese(II) oxide ($MnO$), manganese(III) oxide ($Mn_2O_3$), manganese dioxide ($MnO_2$), trimanganese tetroxide ($Mn_3O_4$), manganese hydroxide ($Mn(OH)_2$), manganese oxyhydroxide ($MnOOH$), manganese chloride ($MnCl_2$), manganese carbonate ($MnCO_3$), manganese sulfate ($MnSO_4$), manganese nitrate ($Mn(NO_3)_2$) and manganese acetate ($Mn(CH_3COO)_2$); one or more selected from the group consisting of manganese oxide, manganese dioxide, trimanganese tetroxide, manganese hydroxide and manganese acetate; one or more selected from the group consisting of manganese dioxide, trimanganese tetroxide and manganese hydroxide; or trimanganese tetroxide.

For example, the iron source is one or more selected from the group consisting of iron(II) oxide ($FeO$), iron(III) oxide ($Fe_2O_3$), triiron) tetroxide ($Fe_3O_4$), iron(II) hydroxide ($Fe(OH)_2$), iron(III) hydroxide ($Fe(OH)_3$), iron(II) chloride ($FeCl_2$), iron(III) chloride ($FeCl_3$) and iron carbonate ($FeCO_3$). The iron source is preferably one or more selected from the group consisting of iron(III) hydroxide, iron(II) hydroxide, iron(III) chloride and iron(II) chloride, and is more preferably iron(III) chloride.

For example, the cobalt source is one or more selected from the group consisting of cobalt(II) oxide ($CoO$), cobalt(IV) oxide ($CoO_2$), tricobalt tetroxide ($Co_3O_4$), cobalt hydroxide ($Co(OH)_2$), cobalt oxyhydroxide ($CoOOH$), cobalt chloride ($CoCl_2$), cobalt carbonate ($CoCOs$), cobalt sulfate ($CoSO_4$), cobalt nitrate ($Co(NO_3)_2$) and cobalt acetate ($Co(CH_3COO)_2$). The cobalt source is preferably one or more selected from the group consisting of cobalt(II) oxide, cobalt(IV) oxide and tricobalt tetroxide, more preferably tricobalt tetroxide.

For example, the nickel source is one or more selected from the group consisting of nickel(II) oxide ($NiO$), nickel(IV) oxide ($NiO_2$), trinickel tetroxide ($Ni_3O_4$), nickel hydroxide ($Ni(OH)_2$), nickel oxyhydroxide ($NiOOH$), nickel chloride ($NiCl_2$), nickel carbonate ($NiCOs$), nickel sulfate ($NiSO_4$), nickel nitrate ($Ni(NO_3)_2$) and nickel acetate ($Ni(CH_3COO)_2$). The nickel source is preferably one or more selected from the group consisting of nickel(II) oxide, nickel(IV) oxide and nickel hydroxide, more preferably nickel(II) oxide.

For example, the copper source is one or more selected from the group consisting of copper (II) oxide ($CuO$), copper (IV) oxide ($CuO_2$), tricopper tetroxide ($Cu_3O_4$), copper hydroxide ($Cu(OH)_2$), copper oxyhydroxide ($CuOOH$), copper chloride ($CuCl_2$), copper carbonate ($CuCOs$), copper sulfate ($CuSO_4$), copper nitrate ($Cu(NO_3)_2$) and copper acetate ($Cu(CH_3COO)_2$).

The raw material composition contains a solvent and is only required to be in a state in which hydrated zirconia, a stabilizing element source and a transition metal element source are dispersed in the solvent. The raw material composition may be regarded as what is called a hydrated zirconia solution, as an aqueous hydrated zirconia solution, or as a hydrated zirconia slurry.

The solvent may be any solvent in which hydrated zirconia can be dispersed, and may be at least one of a polar solvent and a nonpolar solvent. The solvent is preferably at least one of an alcohol and water, more preferably at least one of ethanol and water, and still more preferably water. The water contained in the raw material composition is, for example, at least one of pure water and ion-exchanged water. Furthermore, in the drying step, a solution containing hydrated zirconia obtained by one or more selected from the group consisting of hydrolysis, coprecipitation and neutralization of a zirconium salt and a solvent therefor may be used as the hydrated zirconia and the solvent.

Any method for preparing the raw material composition may be used as long as the hydrated zirconia, the stabilizing element source, and the transition metal element source are mixed uniformly. Examples thereof include (1) a method in which hydrated zirconia, a stabilizing element source, a transition metal element source and a solvent are mixed; (2) a method in which a hydrated zirconia solution is mixed with a stabilizing element source and a transition metal element source; (3) a method in which a hydrated zirconia solution, a solution containing a stabilizing element source and a solution containing a transition metal element source are mixed; (4) a method in which a hydrated zirconia solution, a solution containing a stabilizing element source, and a transition metal element source are mixed; and (5) a method in which a hydrated zirconia solution, a stabilizing element source, and a solution containing a transition metal element source are mixed. The method for preparing the raw material composition is preferably a preparation method including a step of mixing a hydrated zirconia solution, a solution containing a transition metal element source and a stabilizing element source, or a preparation method including a step of mixing a hydrated zirconia solution obtained by hydrolyzing a zirconium salt, a solution containing a transition metal element source, and a stabilizing element source, because the dispersibility of the transition metal element source tends to be higher.

**EP 4 470 991 A1**

Any drying method in the drying step may be used as long as the solvent and water of hydration of the hydrated zirconia can be removed from the raw material composition. The drying conditions include the following conditions.

Drying atmosphere: air atmosphere, preferably air circulating atmosphere
Drying temperature: 150°C or higher, 160°C or higher or 180°C or higher, and 210°C or lower, 200°C or lower or 190°C or lower

The drying time of the raw material composition may be appropriately set in accordance with the amount of the raw material composition to be subjected to the drying step and the characteristics of a drying oven, and is, for example, 5 hours or more or 10 hours or more, and 75 hours or less or 50 hours or less.

For example, preferred drying conditions are described below.

Dry atmosphere: air atmosphere
Drying temperature: 160°C to 200°C

The production method of the present embodiment includes a step of heat-treating the dry powder at a temperature lower than the sintering temperature to provide a calcined powder (hereinafter, also referred to as a "powder calcination step"). When the powder calcination step is performed, the stabilizing element efficiently forms a solid solution with zirconia. In addition, by subjecting the powder to such a heat history before forming the powder into a green body (green compact), aggregation of the transition metal element in the heat treatment after forming is inhibited.

In the powder calcination step, the dried composition is required to be heat-treated at a heat treatment temperature lower than the sintering temperature. The heat treatment temperature in the heat treatment is required to be any temperature that promotes the formation of a solid solution of the stabilizing element with zirconia. Any temperature lower than the sintering temperature may be used in accordance with the desired BET specific surface area. A higher heat treatment temperature tends to result in a lower BET specific surface area. Examples of such a heat treatment temperature can include 1,200°C or lower, lower than 1,200°C, and 1,150°C or lower. The heat treatment temperature is preferably 1,000°C or higher, 1,025°C or higher or 1,050°C or higher, because of further promotion of the formation of a solid solution of the stabilizing element with zirconia. The heat treatment temperature is preferably 1,025°C or higher, 1,075°C or higher or 1,100°C or higher, because local aggregation of the transition metal element is less likely to occur during sintering.

The heat treatment conditions other than the heat treatment temperature is required to be set so as to promote the formation of a solid solution of the stabilizing element with zirconia. Examples of the heat treatment conditions are described below.

Heat treatment atmosphere: oxidizing atmosphere, preferably air atmosphere
Heat treatment temperature: 1,000°C or higher, more than 1,000°C, 1,025°C or higher, 1,050°C or higher or 1,100°C or higher, and
1,200°C or lower or 1,150°C or lower

The air atmosphere is, for example, a nitrogen atmosphere that is mainly composed of nitrogen and oxygen and that has an oxygen concentration of 18% to 23% by volume, and may contain moisture.

The heat treatment time may be appropriately adjusted in accordance with the amount of the dry powder to be subjected to heat treatment and the characteristics of a heat treatment furnace to be used. For example, the heat treatment time may be 30 minutes or more or 1 hour or more, and 10 hours or less or 5 hours or less.

Preferred examples of the heat treatment conditions are described below.

Heat treatment atmosphere: air atmosphere
Heat treatment temperature: 1,075°C or higher and 1,150°C or lower, or 1,100°C or higher and 1,150°C or lower

The calcined powder obtained by the powder calcination step may be used as the powder of the present embodiment.

The production method of the present embodiment may include at least one of a step of grinding the calcined powder (hereinafter, also referred to as a "grinding step") and a step of granulating the calcined powder to provide a granulated powder (hereinafter, also referred to as a "granulation step"), as necessary.

In the grinding step, the calcined powder is ground. This makes it possible to adjust the particle size of the powder. For the grinding, any method that will result in a desired particle size of the calcined powder can be appropriately used. The grinding is only required to be at least one of dry grinding and wet grinding. In view of high grinding efficiency, the grinding is preferably performed by wet grinding, by grinding using one or more selected from the group consisting of a vibration mill, a ball mill, and a bead mill, by grinding using a ball mill and a bead mill, or using a ball mill.

The grinding time may be appropriately set in accordance with the amount of the calcined powder to be subjected to the grinding step, and the grinding method. Longer grinding times result in smaller particle sizes until equilibrium is reached.

Instead of or in addition to the drying step, the calcined powder and an additive component source may be mixed in at least one of the grinding step and the granulation step, or the calcined powder and the additive component source may be mixed in the grinding step.

In the case of producing a powder containing an additive component such as alumina, at least one of the additive component and its precursor (hereinafter also referred to as the "additive component source", and when the additive component source is alumina or the like, it is also referred to as an "alumina source" or the like) may be mixed in one or more selected from the group consisting of the drying step, the powder calcination step, the grinding step and the granulation step. It is preferable to mix the additive component source with at least one of the raw material composition to be subjected to the drying step and the calcined powder to be subjected to the grinding step. It is more preferable to mix the additive component source with the calcined powder to be subjected to the grinding step.

Examples of the additive component source can include the additive component, its hydrate and its sol, and one or more selected from the group consisting of a hydroxide, a halide, a sulfate, a nitrate and an acetate containing one or more selected from the group consisting of aluminum (Al), silicon (Si) and germanium (Ge).

For example, the alumina source is one or more selected from the group consisting of alumina, hydrated alumina, alumina sol, aluminum hydroxide, aluminum chloride, aluminum nitrate and aluminum sulfate, and is preferably alumina.

It is sufficient that the amount of the additive component source contained in the raw material composition be equivalent to the amount of the additive component described above, and, for example, 0 mass% or more, more than 0 mass%, 0.005 mass% or more, 0.01 mass% or more or 0.03 mass% or more, and less than 0.2 mass%, less than 0.15 mass%, less than 0.1 mass% or 0.08 mass% or less. For example, the amount of the additive component is preferably 0 mass% or more and less than 0.2 mass%, 0 mass% or more and less than 0.1 mass% or more than 0 mass% and 0.08 mass% or less.

The amount of the additive component source may be determined as the proportion [mass%] of the additive component [g] on an oxide basis in the total [g] of zirconia, the stabilizing element on an oxide basis, and the metal element on an oxide basis in the raw material composition.

In the granulation step, the calcined powder (the calcined powder after grinding when the grinding step is included) is granulated to provide a granulated powder. This makes it possible to control the flowability of the powder, and further improves the formability of the powder. The granulation may be performed by any granulation method as long as the powder slowly aggregates into a granulated powder, such as a spray granulation method.

In the spray granulation method, it is sufficient to disperse the calcined powder (the calcined powder after grinding when the grinding step is included) in a solvent to prepare a slurry and granulate the slurry. If necessary, the slurry may contain the above-mentioned binder in order to provide a granulated powder with the desired formability.

<Calcined Body · Sintered Body>

The powder of the present embodiment can be used as a precursor of at least one of a calcined body and a sintered body.

The calcined body is obtained by a method for producing a calcined body, the method including a step of calcining a green body containing the powder of the present embodiment (hereinafter, also referred to as a "calcination step"). The sintered body is obtained by a method for producing a sintered body, the method including a step of sintering at least one of a green body containing the powder of the present embodiment and a calcined body obtained by calcining the green body (hereinafter also referred to as a "sintering step").

The green body to be subjected to at least one of the calcination step and the sintering step (hereinafter also referred to as a "calcination step or the like") is a green body containing the powder of the present embodiment or a green body mainly composed of the powder of the present embodiment, preferably a green body composed of the powder of the present embodiment.

The shape of the green body may be one or more selected from the group consisting of a circular plate shape, a cubic shape, a rectangular parallelepiped shape, a polyhedral shape, a substantially polyhedral shape, a cylindrical shape and a conical shape, or may be any other shape according to the purpose and use.

The measured density of the green body is, for example, 2.4 g/cm$^3$ or more or 3.1 g/cm$^3$ or more, and 3.7 g/cm$^3$ or less or 3.5 g/cm$^3$ or less. For example, the measured density of the green body to be subjected to the calcination step or the like is 2.4 g/cm$^3$ or more and 3.7 g/cm$^3$ or less, or 3.2 g/cm$^3$ or more and 3.5 g/cm$^3$ or less.

The green body can be produced by a production method including a step of forming the powder of the present embodiment. A forming method may be any forming method in which the powder of the present embodiment can be formed into a green compact. The forming method is, for example, one or more selected from the group consisting of uniaxial pressing, cold isostatic pressing (hereinafter also referred to as "CIP") treatment, slip casting, sheet forming, slip casting molding and injection molding. One or more selected from the group consisting of slip casting, injection molding, uniaxial pressing, and CIP treatment are preferred. For simplicity, the forming method is preferably at least one of uniaxial pressing and CIP treatment, more preferably a method in which the powder of the present embodiment is uniaxially pressed and the resulting primary green body is subjected to CIP treatment. The pressure in the uniaxial pressing is, for example, 15 MPa or more and 150 MPa or less. The pressure in CIP is, for example, 90 MPa or more and 400 MPa or less.

The calcined body may be a composition in a state in which the green body has been heat-treated at a temperature lower than the sintering temperature, the composition having a certain shape and composed of fused particles of the powder of the present embodiment. The calcined body obtained from the powder of the present embodiment is less susceptible to temperature variations in a sintering furnace than a calcined body obtained from a conventional powder. For this reason, even when a plurality of calcined bodies are simultaneously sintered, the difference in color tone between the resulting sintered bodies is likely to be small. For example, the color tone difference in chroma C* between sintered bodies obtained by sintering calcined bodies in the same lot is 0 or more and 0.1 or less, 0 or more and 0.08 or less, more than 0 and 0.08 or less or 0.01 or more and 0.07 or less.

The measured density of the calcined body is, for example, 2.3 g/cm$^3$ or more or 3.0 g/cm$^3$ or more, and 3.6 g/cm$^3$ or less or 3.4 g/cm$^3$ or less. For example, the measured density of the calcined body is preferably 3.0 g/cm$^3$ or more and 3.4 g/cm$^3$ or less, or 3.3 g/cm$^3$ or more and 3.4 g/cm$^3$ or less.

The Vickers hardness of the calcined body is, for example, 20 HV (= kgf/mm$^2$) or more, 25 HV or more, 30 HV or more or 50 HV or more, and 70 HV or less, 65 HV or less or 60 HV or less. The Vickers hardness is, for example, 50 HV or more and 70 HV or less, 50 HV or more and 65 HV or less or 50 HV or more and 60 HV or less because the calcined body has workability suitable for CAD/CAM processing for dental prosthetic materials.

The crystal phase of the calcined body is preferably at least one of the tetragonal phase and the cubic phase as a main phase.

Any calcination method may be used as long as a calcined body having desired properties is obtained. The following method and conditions can be exemplified.

Calcination atmosphere: an atmosphere other than a reducing atmosphere, preferably an oxidizing atmosphere, more preferably an air atmosphere
Calcination temperature: 800°C or higher, 900°C or higher or 950°C or higher, and 1,200°C or lower, 1,150°C or lower or 1,100°C or lower
Rate of temperature increase: 10 °C/hour or more or 30 °C/hour or more, and 120 °C/hour or less or 80 °C/hour or less

The holding time at the calcination temperature (hereinafter also referred to as a "calcination time") may be appropriately adjusted in accordance with the size and amount of green body to be subjected to calcination and the characteristics of the calcination furnace, and is required to be, for example, 0.5 hours or more or 1 hour or more, and 7 hours or less or 3 hours or less.

Preferred examples of the calcination conditions are described below.

Calcination atmosphere: air atmosphere
Calcination temperature: 900°C or higher and 1,100°C or lower

A sintered body is obtained by sintering at least one of the green body and the calcined body (hereinafter also referred to as a "green body or the like").

The total light transmittance of the sintered body may be any value as long as a desired color tone can be visually recognized. For example, the total light transmittance is 10% or more, 15% or more or 25% or more, and 40% or less, 35% or less or 30% or less.

The color tone of the sintered body may be a desired color tone. The color tone visually recognized varies depending on the translucency. For example, one or more colors selected from the group consisting of yellow-based, green-based, gray-based, and blue-based colors can be exemplified. For example, as a yellow-based color tone in the above-described range of the total light transmittance, a color tone satisfying the following L*, a*, and b* is exemplified.

L*: 55 or more, 60 or more or 63 or more, and
85 or less, 80 or less or 77 or less
a*: -5 or more, -4 or more or -3 or more, and
7 or less, 6 or less or 5 or less
b*: 5 or more, 6 or more or 7 or more, and
35 or less, 30 or less or 25 or less

The three-point bending strength of the sintered body is, for example, 550 MPa or more, 600 MPa or more or 800 MPa or more, and 1,250 MPa or less, less than 1,200 MPa, less than 1,100 MPa or 1,000 MPa or less. The three-point bending strength is preferably 600 MPa or more and 1,200 MPa or less because the sintered body can be used as a dental prosthetic material.

Regarding a sintering method, any sintering method can be applied as long as the sintering of the green body or the like proceeds. One or more sintering methods selected from the group consisting of pressure sintering, vacuum sintering, and pressureless sintering may be used. When a sintered body suitable for a dental prosthetic material is produced, pressureless sintering is preferred. By the pressureless sintering, a pressureless sintered body is obtained.

Examples of the conditions for the pressureless sintering are described below.

Heat treatment atmosphere: an atmosphere other than a reducing atmosphere, preferably an oxidizing atmosphere, more preferably an air atmosphere
Heat treatment temperature: higher than 1,200°C, 1,300°C or higher or 1,400°C or higher, and 1,600°C or lower, 1,550°C or lower or 1,500°C or lower

The holding time at the heat treatment temperature may be freely set in accordance with the size and amount of green body to be sintered, the heat treatment temperature and the characteristics of the sintering furnace, and is, for example, 30 minutes or more or 1 hour or more, and 5 hours or less, 3 hours or less or 2.5 hours or less.

The rate of temperature increase to the heat treatment temperature is, for example, 50 °C/hour or more, 100 °C/hour or more or 150 °C/hour or more, and 800 °C/hour or less or 700 °C/hour or less.

When sintering is performed using a sintering furnace in which sintering can be performed in a short time, the holding time at the heat treatment temperature is, for example, 1 minute or more or 5 minutes or more, and 1 hour or less or 30 minutes or less. In this case, the rate of temperature increase to the heat treatment temperature is, for example, 30 °C/minute or more or 50 °C/minute or more, and 300 °C/minute or 250 °C/minute.

<Appendix>

The gist of the present disclosure may be regarded as any one or more selected from the following groups [1'] to [14'].

[1'] A powder of zirconia, the powder containing a stabilizing element and a transition metal element, in which when a disk-shaped green body obtained by filling 3.0 g of the powder into a mold having a diameter of 25 mm, performing uniaxial pressing at a pressure of 49 MPa and then performing CIP treatment at a pressure of 196 MPa is calcined under the following conditions to provide a calcined body, a shrinkage percentage determined from the following formula is less than 4.0%:

calcination temperature: 1,000°C
calcination time: 1 hour
rate of temperature increase: 50 °C/hour,
calcination atmosphere: air atmosphere,
rate of temperature decrease: 300 °C/hour and

$$\text{shrinkage percentage [\%]} = \{(25 - \text{diameter of calcined body}) \text{ [mm]}/25 \text{ [mm]}\} \times 100 \quad (1).$$

[2'] The powder described in [1'], in which the transition metal element is at least one of a 3d transition metal element and a 4d transition metal element other than zirconium.
[3'] The powder described in [1'], in which the transition metal element is nickel, cobalt, manganese or iron.
[4'] The powder described in [1'], in which the transition metal element is iron.
[5'] The powder described in any one of [1'] to [4'], in which the amount of the transition metal element contained is more than 0 mass%, 0.01 mass% or more or 0.04 mass% or more.
[6'] The powder described in any one of [1'] to [5'], in which the stabilizing element is yttrium (Y).
[7'] The powder described in any one of [1'] to [6'], in which the amount of the stabilizing element contained is 3.5 mol% or more and 5.5 mol% or less.
[8'] The powder described in any one of [1'] to [7'], in which the powder has a BET specific surface area of 8 m$^2$/g or more and 15 m$^2$/g or less.
[9'] The powder described in any one of [1'] to [8'], in which the powder has a bulk density of 1.10 g/cm$^3$ or more and 1.40 g/cm$^3$ or less.

[10'] The powder described in any one of [1'] to [9'], in which the powder is a granulated powder.

[11'] The powder described in any one of [1'] to [10'], in which in a frequency distribution of element ratios of the transition metal element/zirconium plotted at intervals of 0.005, a difference between a minimum value and a maximum value of the transition metal element/zirconium is less than 0.25.

[12'] A method for producing the powder described in [1'] to [11'], including a step of drying a composition containing hydrated zirconia, a stabilizing element source, a transition metal element source and a solvent in an air atmosphere at a drying temperature of 160°C or higher and 200°C or lower to provide a dry powder, and a step of subjecting the dry powder to heat treatment at 1,200°C or lower to provide calcined powder, in which the stabilizing element source is an yttrium source, the transition metal element source is a salt or compound containing at least one of the 3d transition metal element and the 4d transition metal element other than zirconium, and the solvent is water.

[13'] A green body containing the powder described in any one of [1'] to [11'].

[14'] A method for producing a calcined body, including a step of calcining the green body described in [13'].

[15'] A method for producing a sintered body, including a step of sintering at least one of a green body containing the powder described in any one of [1'] to [11'] and a calcined body obtained by calcining the green body.

EXAMPLES

The present disclosure will be described in detail below with reference to examples. However, the present disclosure is not limited to these examples.

(Composition Analysis)

A composition was determined by ICP analysis. As a pretreatment for the analysis, a sample powder was heat-treated in an air atmosphere at 1,000°C for 1 hour.

(BET Specific Surface Area)

The BET specific surface area was measured by a five-point BET method in accordance with JIS R 1626 using an automated specific surface area measuring device (device name: Tristar II 3020, manufactured by Shimadzu Corporation). The measurement conditions are described below.

Adsorption medium: $N_2$
Adsorption temperature: -196°C
Pretreatment conditions: degassing treatment in air atmosphere at 250°C for 1 hour or more

(Average Particle Size)

The average particle size was measured by particle size distribution measurement by a laser diffraction/scattering method using a Microtrac particle size distribution analyzer (device name: MT3300EXII, manufactured by Microtrac Bell). The measurement conditions are described below.

Light source: semiconductor laser (wavelength: 780 nm)
Voltage: 3 mW
Measurement sample: ground slurry
Refractive index of zirconia: 2.17
Refractive index of solvent (water): 1.333
Calculation mode: HRA

As a pretreatment, a sample powder was suspended in distilled water to prepare a slurry. Then the slurry was subjected to dispersion treatment for three minutes with an ultrasonic homogenizer (device name: US-150T, manufactured by Nihonseiki Kaisha Ltd.).

(Measured Density)

A measured density [g/cm$^3$] was calculated from the mass [g] relative to the volume of a sample [cm$^3$]. For the mass, the mass determined by weighing the sample was used. For the volume, the volume determined by shape measurement was used for a green body and a calcined body. For a sintered body, the volume obtained by Archimedes' method according to JIS R 1634 may be used. In Archimedes' method, ion-exchanged water was used as a solvent, and pretreatment was performed by a boiling method.

(Shrinkage Percentage)

The shrinkage percentage of a powder was measured using a disk-shaped calcined body having a diameter of 25 mm or less and a thickness of 2 ± 0.5 mm. The calcined body was produced by filling 3.0 g of the powder into a mold having a diameter of 25 mm, performing uniaxial pressing at a pressure of 49 MPa, then performing CIP treatment at a pressure of 196 MPa to provide a disk-shaped green body and calcining the green body under the following conditions.

Calcination temperature: 1,000°C
Calcination time: 1 hour
Rate of temperature increase: 50 °C/hour
Calcination atmosphere: air atmosphere
Rate of temperature decrease: 300 °C/hour

Shrinkage percentage [%] = {(25 - diameter of calcined body) [mm]/25 [mm]} × 100     (1)

As the diameter of the calcined body, the average value of values obtained by measuring the length of the disk in the diametrical direction at four points using a vernier caliper was used.

(Total Light Transmittance)

The total light transmittance was measured by a method in accordance with JIS K 7361-1 with a hazemeter (device name: NDH4000, manufactured by Nippon Denshoku Industries Co., Ltd.) using illuminant D65 as a light source. A measurement sample used was a disk-shaped sintered body having a thickness of 1.0 ± 0.1 mm and having been polished on both sides so as to have a surface roughness of Ra ≤ 0.02 μm.

(Color Tone)

A color tone was measured in SCI mode with a spectrocolorimeter (device name: CM-700d, manufactured by Konica Minolta Inc.) using illuminant D65 as a light source. A measurement sample used was a disk-shaped sintered body having a thickness of 1.0 ± 0.1 mm and having been polished on both sides so as to have a surface roughness of Ra ≤ 0.02 μm. The measurement sample was placed on a black plate, and both surfaces after polishing were used as evaluation surfaces, and the color tones (L*, a*, and b*) were measured (what is called black back measurement). Furthermore, chroma C* was calculated from a* and b*.

(Three-Point Bending Strength)

The three-point bending strength was measured by a method according to JIS R 1601. The measurement sample had a pillar shape with a width of 4 mm, a thickness of 3 mm and a length of 45 mm. The measurement was performed with a span of 30 mm under a load applied in the horizontal direction of the measurement sample.

(Vickers Hardness)

The Vickers hardness was measured with a Vickers tester (device name: Q30A, manufactured by Qness). An indenter was statically pressed onto the surface of a measurement sample under the following conditions, and the diagonal length of an indentation mark formed on the surface of the measurement sample was measured. The diagonal length thus obtained was used to calculate the Vickers hardness from the above formula.

Measurement sample: a disk shape having a thickness of 3.0 ± 0.5 mm
Measurement load: 1 kgf

Prior to the measurement, a calcined body in which the measurement surface was polished by 0.1 mm with #800 waterproof abrasive paper was used as a measurement sample.

(Element Frequency Distribution)

The element frequency distribution was determined from an electron probe microanalysis (EPMA) spectrum obtained under the following conditions using a wavelength-dispersive electron probe microanalyzer (device name: EPMA1610, manufactured by Shimadzu Corporation) or a field-emission wavelength-dispersive electron probe microanalyzer (device name: JXA-iHP200F, manufactured by JEOL Ltd.).

Accelerating voltage: 15 kV
Illumination current: 50 nA
Probe diameter: 1 $\mu$m
Acquisition time: 50 msec
Magnification: 5,000$\times$
Analysis area: 45.32 $\mu$m $\times$ 45.32 $\mu$m to 51.20 $\mu$m $\times$ 51.20 $\mu$m

A powder sample was embedded in epoxy resin and then cut by ion milling. The cross section of the powder exposed after cutting was used as an observation surface, and gold (Au) was vapor-deposited onto the surface to prepare a measurement sample.
For the EPMA spectrum, a SEM observation image was divided into 50,000 to 66,000 regions, and each divided region was used as a measurement point. M/Zr was calculated from the intensities of characteristic X-rays of zirconium and a transition metal element (M) in the EPMA spectrum at each measurement point. The resulting M/Zr frequencies were plotted as described above to obtain an element frequency distribution. From the resulting element frequency distribution, an M/Zr range, the minimum value of M/Zr, the maximum value of M/Zr, a high metal frequency and a low metal frequency were determined.

(Standard Sample)

As a standard sample, a sintered body was produced using a commercially available zirconia powder. That is, 3.0 g of a commercially available zirconia powder (product name: Zpex, manufactured by Tosoh Corporation) was weighed, filled into a mold having a diameter of 25 mm and subjected to uniaxial pressing at a pressure of 19.6 MPa and then CIP treatment at a pressure of 196 MPa, thereby providing a disk-shaped green body.
The resulting green body was calcined under the following conditions to provide a calcined body.

Calcination temperature: 1,000°C
Calcination time: 1 hour
Rate of temperature increase: 50 °C/hour
Calcination atmosphere: air atmosphere
Rate of temperature decrease: 300 °C/hour

The resulting calcined body was sintered under the following conditions to provide a sintered body having a total light transmittance of 42%. This sintered body was used as a standard sample.

Sintering method: pressureless sintering
Sintering temperature: 1,450°C
Sintering time: 2 hours
Rate of temperature increase: 600 °C/hour
Sintering atmosphere: air atmosphere

Example 1

Yttrium chloride was added to and mixed with 5 L of an aqueous solution of hydrated zirconia obtained by subjecting an aqueous solution of zirconium oxychloride to hydrolysis in such a manner that the yttrium concentration was 4.0 mol%, thereby preparing a mixed aqueous solution. After mixing, an aqueous solution of iron(III) chloride with an $FeCl_3$ concentration of 45 mass% was added to and mixed with the mixed aqueous solution in such a manner that the iron concentration was 0.2 mass% in terms of $Fe_2O_3$, thereby preparing a raw material composition (aqueous sol solution).
The raw material composition was dried at 180°C in an air atmosphere to remove water. The resulting dry powder was fired at 1,125°C in an air atmosphere to provide a calcined powder (a powder of yttrium-containing zirconia having an amount of yttrium of 4.0 mol%, the powder containing 0.2 mass% of iron).
199.9 g of the resulting calcined powder, 0.1 g of an $\alpha$-alumina powder, and pure water were mixed and ground in a ball mill using zirconia balls having a diameter of 2 mm as grinding media, thereby preparing a slurry. An acrylic resin was added to the slurry in such a manner that the proportion by mass of the binder based on the mass of the powder in the slurry was 3 mass%. Thereafter, the mixture was spray-dried at 180°C in an air atmosphere to produce a granulated powder composed of a powder of yttria-containing zirconia having an amount of yttrium of 4.0 mol%, the granulated powder containing 3 mass% of the acrylic resin, 0.2 mass% of iron and 0.05 mass% of alumina. This granulated powder was defined as a powder of the present example.

3.0 g of the resulting granulated powder was filled into a mold having a diameter of 25 mm, subjected to uniaxial pressing at a pressure of 49 MPa and then CIP treatment at a pressure of 196 MPa, thereby providing a disk-shaped green body (green compact).

The measured density of the resulting green body was 3.32 g/cm$^3$. The green body was calcined under the following conditions to provide a calcined body of the present example.

    Calcination temperature: 1,000°C
    Calcination time: 1 hour
    Rate of temperature increase: 50 °C/hour
    Calcination atmosphere: air atmosphere
    Rate of temperature decrease: 300 °C/hour

Example 2

A granulated powder composed of a powder of yttria-containing zirconia having an amount of yttrium of 4.0 mol%, the granulated powder containing 3 mass% of the acrylic resin, 0.05 mass% of cobalt and 0.05 mass% of alumina, was produced in the same manner as in Example 1, except that tricobalt tetroxide was added instead of iron in such a manner that the cobalt concentration was 0.05 mass% in terms of $Co_3O_4$, and the calcination temperature was 1,140°C. This granulated powder was defined as a powder of the present example.

A green body and a calcined body were produced in the same manner as in Example 1, except that the powder of the present example was used. The measured density of the resulting green body was 3.30 g/cm$^3$.

Example 3

A granulated powder composed of a powder of yttria-containing zirconia having an amount of yttrium of 4.0 mol%, the granulated powder containing 3 mass% of the acrylic resin, 0.05 mass% of manganese and 0.05 mass% of alumina, was produced in the same manner as in Example 1, except that trimanganese tetroxide ($Mn_3O_4$) was added instead of iron in such a manner that the manganese concentration was 0.05 mass% in terms of $Mn_3O_4$, and the calcination temperature was 1,140°C. This granulated powder was used as a powder of the present example.

A green body and a calcined body were produced in the same manner as in Example 1, except that the powder of the present example was used. The measured density of the resulting green body was 3.30 g/cm$^3$.

Example 4

A granulated powder composed of a powder of yttria-containing zirconia having an amount of yttrium of 4.0 mol%, the granulated powder containing 3 mass% of the acrylic resin, 0.05 mass% of nickel and 0.05 mass% of alumina, was produced in the same manner as in Example 1, except that nickel oxide (NiO) was added instead of iron chloride in such a manner that the nickel concentration was 0.05 mass% in terms of NiO, and the calcination temperature was 1,140°C. This granulated powder was defined as a powder of the present example.

A green body and a calcined body were produced in the same manner as in Example 1, except that the powder of the present example was used. The measured density of the resulting green body was 3.30 g/cm$^3$.

Comparative Example 1

A raw material composition was prepared in the same manner as in Example 1, except that aqueous solution of iron(III) chloride was not added, and the calcination temperature was 1,175°C. Then the raw material composition was dried and calcined to provide a calcined powder.

The resulting calcined powder was mixed with iron(III) hydroxide oxide in a ball mill to provide a powder of yttria-containing zirconia having an amount of yttrium of 4.0 mol%, the powder containing 0.2 mass% of iron.

A granulated powder composed of a powder of yttria-containing zirconia having an amount of yttrium of 4.0 mol%, the granulated powder containing 3 mass% of the acrylic resin, 0.2 mass% of iron and 0.05 mass% of alumina, was produced in the same manner as in Example 1, except that 199.9 g of the resulting power was used. This granulated powder was defined as a powder of the present comparative example.

A green body and a calcined body were produced in the same manner as in Example 1, except that the powder of the present comparative example was used. The measured density of the resulting green body was 3.33 g/cm$^3$.

Comparative Example 2

A powder of yttria-containing zirconia having an amount of yttrium of 4.0 mol%, the powder containing 3 mass% of the acrylic resin, 0.05 mass% of cobalt and 0.05 mass% of alumina, was produced in the same manner as in Comparative Example 1, except that tricobalt tetroxide ($Co_3O_4$) was used instead of iron(III) hydroxide oxide in such a manner that the cobalt content was 0.05 mass% in terms of $Co_3O_4$. This powder was defined as a powder of the present comparative example.

A green body and a calcined body were produced in the same manner as in Example 1, except that the powder of the present comparative example was used. The measured density of the resulting green body was 3.30 g/cm$^3$.

Comparative Example 3

A powder of yttria-containing zirconia having an amount of yttrium of 4.0 mol%, the powder containing 3 mass% of the acrylic resin, 0.05 mass% of manganese and 0.05 mass% of alumina, was produced in the same manner as in Comparative Example 1, except that trimanganese tetroxide ($Mn_3O_4$) was used instead of iron(III) hydroxide oxide in such a manner that the manganese content was 0.05 mass% in terms of $Mn_3O_4$. This powder was defined as a powder of the present comparative example.

A green body and a calcined body were produced in the same manner as in Example 1, except that the powder of the present comparative example was used. The measured density of the resulting green body was 3.30 g/cm$^3$.

The evaluation results of the powders of the examples and the comparative examples are shown in Tables 1 and 2.

[Table 1]

| | | $Y_2O_3$ (mol%) | $Al_2O_3$ (wt%) | Transition metal element | | Average particle size ($\mu$m) | BET specific surface area (m$^2$/g) | Average granule size ($\mu$m) | Bulk density (g/cm$^3$) | T+C phase ratio (%) | Shrinkage percentage (%) |
| | | | | Type | Content (wt%) | | | | | | |
|---|---|---|---|---|---|---|---|---|---|---|---|
| | Example 1 | 4.0 | 0.05 | Fe | 0.2 | 0.44 | 10.5 | 47 | 1.27 | 90 | 3.6 |
| | Example 2 | 4.0 | 0.05 | Co | 0.05 | 0.43 | 10.3 | 46 | 1.27 | 90 | 3.5 |
| | Example 3 | 4.0 | 0.05 | Mn | 0.05 | 0.44 | 10.5 | 45 | 1.25 | 90 | 3.6 |
| | Example 4 | 4.0 | 0.05 | Ni | 0.05 | 0.44 | 10.6 | 45 | 1.24 | 90 | 3.5 |
| | Comparative Example 1 | 4.0 | 0.05 | Fe | 0.2 | 0.43 | 10.2 | 47 | 1.29 | 90 | 4.3 |
| | Comparative Example 2 | 4.0 | 0.05 | Co | 0.05 | 0.42 | 10.4 | 45 | 1.29 | 90 | 4.0 |
| | Comparative Example 3 | 4.0 | 0.05 | Mn | 0.05 | 0.43 | 10.4 | 43 | 1.29 | 90 | 4.1 |

The results indicated that the powders of Example 1 and Comparative Example 1 in which iron was contained as the transition metal element, Example 2 and Comparative Example 2 in which cobalt was contained as the transition metal element and Example 3 and Comparative Example 3 in which manganese was contained as the transition metal element had similar physical properties.

In addition, the results reveal that the calcined bodies of the comparative examples have large shrinkage percentages and the densification is further progressed by the calcination, compared with the calcined bodies of the examples. This indicates that in the powders of the examples, thermal shrinkage during the calcination is reduced, compared with the powders of the comparative examples.

[Table 2]

|  | M/Zr range | Maximum value of M/Zr | High metal frequency (%) | Low metal frequency (%) |
|---|---|---|---|---|
| Example 1 | 0.065 | 0.065 | 0.05 | 4.16 |
| Example 2 | 0.105 | 0.120 | 70.93 | 0 |
| Example 3 | 0.105 | 0.105 | 3.90 | 0.01 |
| Example 4 | 0.150 | 0.175 | 95.85 | 0 |
| Comparative Example 1 | 0.250 | 0.250 | 2.67 | 6.54 |

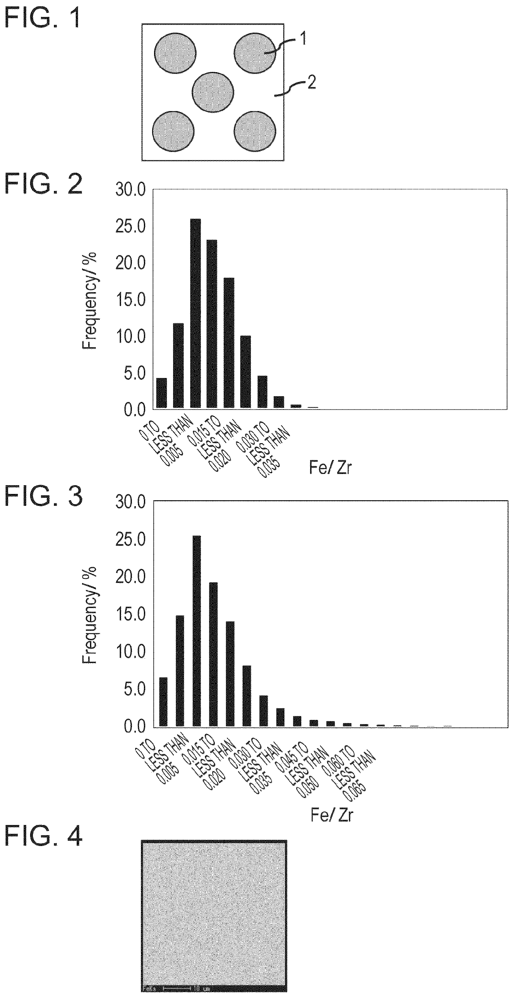

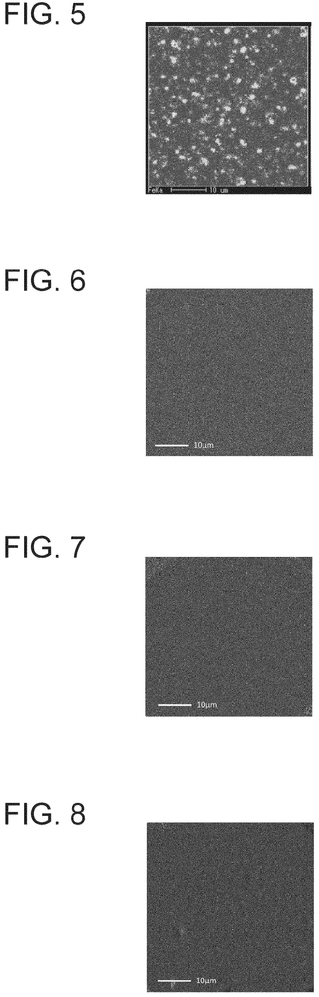

From the above table, it can be seen that the powders of Examples 1 to 4 all have an M/Zr range of less than 0.25, or even 0.2 or less, and that the transition metal elements are uniformly dispersed, compared with the powders of the comparative examples. Elemental mapping (iron mapping) of the transition metal element (iron) in the powder of Example 1 indicates a substantially uniform distribution state (Fig. 4), and it can be seen that there are no aggregated particles of iron. In contrast, in the elemental mapping of the transition metal element (iron mapping) in the powder of Comparative Example 1, many spots of indefinite shapes are observed, indicating the presence of aggregated particles of iron (Fig. 5). It can be seen that, like iron, cobalt (Fig. 6, Example 2), manganese (Fig. 7, Example 3) and nickel (Fig. 8, Example 4) are each uniformly distributed.

The evaluation results of the calcined bodies of the examples and comparative examples are presented in Table 3.

[Table 3]

|  | Measured density (g/cm$^3$) | Vickers hardness (Hv) |
|---|---|---|
| Example 1 | 3.27 | 55 |
| Example 2 | 3.29 | 54 |
| Example 3 | 3.29 | 57 |
| Example 4 | 3.29 | 53 |
| Comparative Example 1 | 3.41 | 76 |
| Comparative Example 2 | 3.31 | 71 |
| Comparative Example 3 | 3.31 | 73 |

From the above table, it can be seen that the Vickers hardness of each of the calcined bodies obtained from the powders of the examples was 65 Hv or less, which is lower than the Vickers hardness of the calcined body obtained from the powder of Comparative Example 1. This indicates that the use of the powders of the examples results in calcined bodies with high workability.

Measurement Example 1 (Production of Sintered Body)

Five calcined bodies for each of the examples and comparative examples were prepared in the same manner as in the examples and comparative examples. The resulting calcined bodies were placed in alumina saggers as illustrated in Fig. 1, placed in a sintering furnace, and sintered under the following conditions to provide five sintered bodies each.

Sintering method: pressureless sintering
Sintering temperature: 1,500°C
Sintering time: 2 hours
Rate of temperature increase: 600 °C/hour
Sintering atmosphere: air atmosphere

The results of the sintered bodies obtained are presented in a table below. The total light transmittance in the table below indicates the proportion of the total light transmittance of the example (or comparative example) relative to the value of the standard sample. Each value in the table below is the average value of five sintered bodies.

[Table 4]

| Calcined body | Sintering shrinkage percentage (%) | Sintered body | | | | |
|---|---|---|---|---|---|---|
| | | Proportion of total light transmittance | Color tone | | | Three-point bending strength (MPa) |
| | | | L* | a* | b* | |
| Example 1 | 21.2 | 0.60 | 67.92 | 3.28 | 19.24 | 1060 |
| Example 2 | 21.2 | 0.31 | 60.25 | 3.46 | -4.49 | 1070 |
| Example 3 | 21.2 | 0.10 | 52.29 | 2.22 | -3.24 | 1080 |
| Example 4 | 21.2 | 0.62 | 68.83 | 2.87 | 10.24 | 1040 |
| Comparative Example 1 | 20.9 | 0.62 | 68.42 | 3.71 | 20.46 | 1070 |
| Comparative Example 2 | 21.3 | 0.31 | 59.70 | 2.79 | -4.95 | 1020 |
| Comparative Example 3 | 21.3 | 0.10 | 51.86 | 1.76 | -2.81 | 1040 |

From the comparison between Example 1 and Comparative Example 1, the comparison between Example 2 and Comparative Example 2, and the comparison between Example 3 and Comparative Example 3, it can be seen that all of the resulting sintered bodies exhibit similar aesthetics and mechanical strength.

The standard deviation of chroma C* of five sintered bodies of each of the examples and comparative examples is presented in a table below.

[Table 5]

| | Standard deviation of C* |
|---|---|
| Example 1 | 0.04 |
| Example 2 | 0.05 |
| Example 3 | 0.06 |
| Example 4 | 0.03 |
| Comparative Example 1 | 0.12 |
| Comparative Example 2 | 0.10 |
| Comparative Example 3 | 0.10 |

The above table indicated that the sintered bodies of Examples have a small standard deviation of C* and a small change in color tone due to the temperature distribution (temperature unevenness) in the sintering furnace as compared with the sintered bodies of Comparative Examples, and that sintered bodies exhibiting similar aesthetic properties can be produced with high reproducibility from the powders and calcined bodies of the examples.

Example 5

A granulated powder composed of a powder of yttria-containing zirconia having an amount of yttrium of 5.2 mol%, the granulated powder containing 3 mass% of the acrylic resin and 0.18 mass% of iron, was produced in the same manner as in Example 1, except that yttrium chloride was added in such a manner that the yttrium concentration was 5.2 mol%, an aqueous solution of iron(III) chloride having an $FeCl_3$ concentration of 45 mass% was added to the mixed aqueous solution in such a manner that the iron concentration was 0.18 mass% in terms of $Fe_2O_3$, and an $\alpha$-alumina powder was not used. This granulated powder was defined as a powder of the present example.

A green body and a calcined body were produced in the same manner as in Example 1, except that the powder of the present example was used. The measured density of the resulting green body was 3.30 g/cm$^3$.

Example 6

A granulated powder composed of a powder of yttria-containing zirconia having an amount of yttrium of 5.2 mol%, the granulated powder containing 3 mass% of the acrylic resin and 0.21 mass% of iron, was produced in the same manner as in Example 1, except that yttrium chloride was added in such a manner that the yttrium concentration was 5.2 mol%, an aqueous solution of iron(III) chloride having an $FeCl_3$ concentration of 45 mass% was added to the mixed aqueous solution in such a manner that the iron concentration was 0.21 mass% in terms of $Fe_2O_3$, and an $\alpha$-alumina powder was not used. This granulated powder was defined as a powder of the present example.

A green body and a calcined body were produced in the same manner as in Example 1, except that the powder of the present example was used. The measured density of the resulting green body was 3.30 $g/cm^3$.

Example 7

A granulated powder composed of a powder of yttria-containing zirconia having an amount of yttrium of 5.2 mol%, the granulated powder containing 3 mass% of the acrylic resin and 0.23 mass% of iron, was produced in the same manner as in Example 1, except that yttrium chloride was added in such a manner that the yttrium concentration was 5.2 mol%, an aqueous solution of iron(III) chloride having an $FeCl_3$ concentration of 45 mass% was added to the mixed aqueous solution in such a manner that the iron concentration was 0.23 mass% in terms of $Fe_2O_3$, and an $\alpha$-alumina powder was not used. This granulated powder was defined as a powder of the present example.

A green body and a calcined body were produced in the same manner as in Example 1, except that the powder of the present example was used. The measured density of the resulting green body was 3.30 $g/cm^3$.

The evaluation results of the resulting powders are presented in Table 6, and the evaluation results of the calcined bodies are presented in Table 7.

Example 8

A granulated powder composed of a powder of yttria-containing zirconia having an amount of yttrium of 5.2 mol%, the granulated powder containing 3 mass% of the acrylic resin and 0.25 mass% of iron, was produced in the same manner as in Example 1, except that yttrium chloride was added in such a manner that the yttrium concentration was 5.2 mol%, an aqueous solution of iron(III) chloride having an $FeCl_3$ concentration of 45 mass% was added to the mixed aqueous solution in such a manner that the iron concentration was 0.25 mass% in terms of $Fe_2O_3$, and an $\alpha$-alumina powder was not used. This granulated powder was defined as a powder of the present example.

A green body and a calcined body were produced in the same manner as in Example 1, except that the powder of the present example was used. The measured density of the resulting green body was 3.30 $g/cm^3$.

The powders of Examples 5 to 8 had an average particle size of 0.43 $\mu$m to 0.45 $\mu$m, a BET specific surface area of 10.6 to 10.9 $m^2/g$, an average granule size of 42 $\mu$m to 46 $\mu$m, a bulk density of 1.27 to 1.29 $g/cm^3$, a T+C phase ratio of 95% and a shrinkage percentage of 3.5%, and all the powders had similar powder physical properties.

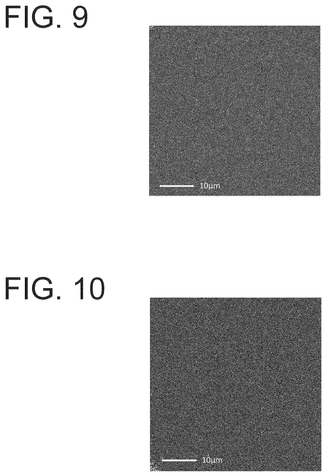

In the powder of Example 8, the M/Zr range (Fe/Zr range) was 0.058, the maximum value of M/Zr was 0.060, the high metal frequency was 0.04%, and the low metal frequency was 0.01%. The elemental mapping of iron in Example 8 is illustrated in Fig. 9. In the calcined bodies of Examples 5 to 9, the transition metal element (iron) was uniformly dispersed, compared with the calcined body of Comparative Example 1.

The evaluation results of the calcined bodies of Examples 5 to 8 are presented in a table below.

[Table 6]

|  | Transition metal element (iron) (wt%) | Measured density ($g/cm^3$) | Shrinkage percentage (%) | Vickers hardness (Hv) |
|---|---|---|---|---|
| Example 5 | 0.18 | 3.30 | 3.5 | 53 |
| Example 6 | 0.21 | 3.30 | 3.5 | 54 |
| Example 7 | 0.23 | 3.30 | 3.5 | 55 |
| Example 8 | 0.25 | 3.30 | 3.5 | 56 |

The above table indicated that the Vickers hardness tended to increase as the amount of the transition metal element increased, but the degree of increase was very small. The influence of the amount of the transition metal element on the measured density and shrinkage percentage was hardly observed.

Measurement Example 2 (Production of Sintered Body)

Sintered bodies were produced and evaluated in the same manner as in Measurement Example 1, except that five calcined bodies for each example were produced in the same manner as in Examples 5 to 8. The results are presented in a table below.

[Table 7]

| Calcined body | Sintering shrinkage rate (%) | Sintered body | | | | |
|---|---|---|---|---|---|---|
| | | Proportion of total light transmittance | Color tone | | | Three-point bending strength (MPa) |
| | | | L* | a* | b* | |
| Example 5 | 21.3 | 0.74 | 66.27 | 2.99 | 19.40 | 810 |
| Example 6 | 21.3 | 0.71 | 65.58 | 3.61 | 20.03 | 810 |
| Example 7 | 21.3 | 0.69 | 65.61 | 3.73 | 19.81 | 830 |
| Example 8 | 21.3 | 0.69 | 64.63 | 3.94 | 19.58 | 810 |

From the above table, it can be seen that the transmittance tends to decrease as the transition metal element content increases, and the color tone tends to become darker due to a decrease in L* and an increase in a*.

The standard deviation of the chroma C* of the five sintered bodies of each of Examples 5 to 8 is presented in a table below together with the evaluation results of Comparative Example 1.

[Table 8]

| | Standard deviation of C* |
|---|---|
| Example 5 | 0.04 |
| Example 6 | 0.05 |
| Example 7 | 0.06 |
| Example 8 | 0.05 |
| Comparative Example 1 | 0.12 |

From the above table, it can be seen that variations in color tone due to the change in transition metal element content are very small, and in particular, the standard deviation of C* is small in Examples 6 to 8 despite the fact that a large amount of the transition metal element (iron) is contained, as compared with Comparative Example 1.

Example 9

A granulated powder composed of a powder of yttria-containing zirconia having an amount of yttrium of 3.0 mol%, the granulated powder containing 3 mass% of the acrylic resin, 0.15 mass% of iron and 0.05 mass% of alumina, was produced in the same manner as in Example 1, except that yttrium chloride was added in such a manner that the yttrium concentration was 3.0 mol%, an aqueous solution of iron(III) chloride having an $FeCl_3$ concentration of 45 mass% was added to the mixed aqueous solution in such a manner that the iron concentration was 0.15 mass% in terms of $Fe_2O_3$, and the dry powder was fired at 1,135°C in an air atmosphere. This granulated powder was defined as a powder of the present example.

A green body and a calcined body were produced in the same manner as in Example 1, except that the powder of the present example was used. The measured density of the resulting green body was 3.31 g/cm$^3$.

Example 10

A granulated powder composed of a powder of yttria-containing zirconia having an amount of yttrium of 4.8 mol%, the granulated powder containing 3 mass% of the acrylic resin and 0.25 mass% of iron, was produced in the same manner as in Example 1, except that yttrium chloride was added in such a manner that the yttrium concentration was 4.8 mol%, an aqueous solution of iron(III) chloride having an $FeCl_3$ concentration of 45 mass% was added to the mixed aqueous solution in such a manner that the iron concentration was 0.25 mass% in terms of $Fe_2O_3$, the dry powder was fired at 1,095°C in an air atmosphere, and an $\alpha$-alumina powder was not used. This granulated powder was defined as a powder of the present example.

A green body and a calcined body were produced in the same manner as in Example 1, except that the powder of the present example was used. The measured density of the resulting green body was 3.27 g/cm$^3$.

Example 11

A granulated powder composed of a powder of yttria-containing zirconia having an amount of yttrium of 5.0 mol%, the granulated powder containing 3 mass% of the acrylic resin and 0.25 mass% of iron, was produced in the same manner as in Example 1, except that yttrium chloride was added in such a manner that the yttrium concentration was 5.0 mol%, an aqueous solution of iron(III) chloride having an $FeCl_3$ concentration of 45 mass% was added to the mixed aqueous solution in such a manner that the iron concentration was 0.25 mass% in terms of $Fe_2O_3$, the dry powder was fired at 1,095°C in an air atmosphere, and an $\alpha$-alumina powder was not used. This granulated powder was defined as a powder of the present example.
A green body and a calcined body were produced in the same manner as in Example 1, except that the powder of the present example was used. The measured density of the resulting green body was 3.27 g/cm$^3$.

Example 12

A granulated powder composed of a powder of yttria-containing zirconia having an amount of yttrium of 5.2 mol%, the granulated powder containing 3 mass% of the acrylic resin and 0.25 mass% of iron, was produced in the same manner as in Example 1, except that yttrium chloride was added in such a manner that the yttrium concentration was 5.2 mol%, an aqueous solution of iron(III) chloride having an $FeCl_3$ concentration of 45 mass% was added to the mixed aqueous solution in such a manner that the iron concentration was 0.25 mass% in terms of $Fe_2O_3$, the dry powder was fired at 1,095°C in an air atmosphere, and an $\alpha$-alumina powder was not used. This granulated powder was defined as a powder of the present example.
A green body and a calcined body were produced in the same manner as in Example 1, except that the powder of the present example was used. The measured density of the resulting green body was 3.27 g/cm$^3$.

Example 13

A granulated powder composed of a powder of yttria-containing zirconia having an amount of yttrium of 5.2 mol%, the granulated powder containing 3 mass% of the acrylic resin and 0.25 mass% of iron, was produced in the same manner as in Example 1, except that yttrium chloride was added in such a manner that the yttrium concentration was 5.2 mol%, an aqueous solution of iron(III) chloride having an $FeCl_3$ concentration of 45 mass% was added to the mixed aqueous solution in such a manner that the iron concentration was 0.25 mass% in terms of $Fe_2O_3$, the dry powder was fired at 1,080°C in an air atmosphere, and an $\alpha$-alumina powder was not used. This granulated powder was defined as a powder of the present example.
A green body and a calcined body were produced in the same manner as in Example 1, except that the powder of the present example was used. The measured density of the resulting green body was 3.26 g/cm$^3$.

Example 14

A granulated powder composed of a powder of yttria-containing zirconia having an amount of yttrium of 5.2 mol%, the granulated powder containing 3 mass% of the acrylic resin, 0.25 mass% of iron and 0.05 mass% of alumina, was produced in the same manner as in Example 1, except that yttrium chloride was added in such a manner that the yttrium concentration was 5.2 mol%, an aqueous solution of iron(III) chloride having an $FeCl_3$ concentration of 45 mass% was added to the mixed aqueous solution in such a manner that the iron concentration was 0.25 mass% in terms of $Fe_2O_3$, and a dry powder was fired in an air atmosphere at 1,100°C. This granulated powder was defined as a powder of the present example.
A green body and a calcined body were produced in the same manner as in Example 1, except that the powder of the present example was used. The measured density of the resulting green body was 3.30 g/cm$^3$.
The powders of Examples 9 to 14 had an average particle size of 0.41 $\mu$m to 0.45 $\mu$m, an average granule size of 43 $\mu$m to 46 $\mu$m and a bulk density of 1.26 to 1.28 g/cm$^3$.
The evaluation results of the powders and calcined bodies of Examples 12 and 13 are presented in a table below together with the evaluation results of Example 8.

[Table 9]

| | Powder | | | | | Calcined body | |
|---|---|---|---|---|---|---|---|
| | $Y_2O_3$ (mol%) | Iron content (wt%) | BET specific surface area ($m^2/g$) | T+C phase ratio (%) | Shrinkage percentage (%) | Measured density ($g/cm^3$) | Vickers hardness (Hv) |
| Example 8 | 5.2 | 0.25 | 10.9 | 95 | 3.5 | 3.30 | 56 |
| Example 12 | 5.2 | 0.25 | 12.5 | 95 | 3.7 | 3.30 | 61 |
| Example 13 | 5.2 | 0.25 | 13.7 | 95 | 3.8 | 3.31 | 65 |

From the above table, it can be seen that as the BET specific surface area of the powders increases, the measured density and Vickers hardness of the resulting calcined bodies increase, and in addition, the increase in Vickers hardness is greater than the increase in the measured density.

The evaluation results of the powders and calcined bodies of Examples 9 to 12 are presented in a table below.

[Table 10]

| | Powder | | | | | Calcined body | |
|---|---|---|---|---|---|---|---|
| | $Y_2O_3$ (mol%) | Iron content (wt%) | BET specific surface area ($m^2/g$) | T+C phase ratio (%) | Shrinkage percentage (%) | Measured density ($g/cm^3$) | Vickers hardness (Hv) |
| Example 9 | 3.0 | 0.15 | 10.5 | 65 | 3.6 | 3.31 | 55 |
| Example 10 | 4.8 | 0.25 | 11.9 | 92 | 3.7 | 3.27 | 61 |
| Example 11 | 5.0 | 0.25 | 12.0 | 93 | 3.7 | 3.27 | 61 |
| Example 12 | 5.2 | 0.25 | 12.5 | 95 | 3.7 | 3.27 | 61 |

It can be seen that the calcined bodies of Examples 10 to 12 have higher Vickers hardness than the calcined bodies of Example 9. Since the difference in Vickers hardness between Example 9 and Examples 10 to 12 is about 6 HV, the difference in Vickers hardness is considered to be due to the influence of the BET specific surface area and the amount of the transition metal (iron content). Furthermore, the Vickers hardness of the calcined bodies of Examples 10 to 12 was substantially the same, and the effect of the amount of the stabilizing elements was not observed.

In the powder of Example 9, the M/Zr range (Fe/Zr range) was 0.115, the maximum value of M/Zr was 0.115, the high metal frequency was 0.06%, and the low metal frequency was 0.45%. The elemental mapping of iron in Example 9 is illustrated in Fig. 10. In the calcined bodies of Examples 9 to 14, the transition metal element (iron) was uniformly dispersed, compared with the calcined bodies of Comparative Example 1.

Measurement Example 3 (Production of Sintered Body)

Sintered bodies were produced and evaluated in the same manner as in Measurement Example 1, except that five calcined bodies for each example were produced in the same manner as in Examples 9 to 14. The results are presented in a table below.

[Table 11]

| Calcined body | Sintering shrinkage rate (%) | Sintered body | | | | |
|---|---|---|---|---|---|---|
| | | Proportion of total light transmittance | Color tone | | | Three-point bending strength (MPa) |
| | | | L* | a* | b* | |
| Example 9 | 21.4 | 0.50 | 69.82 | 4.58 | 20.55 | 1200 |
| Example 10 | 21.5 | 0.67 | 65.73 | 3.60 | 20.37 | 960 |
| Example 11 | 21.5 | 0.71 | 66.15 | 2.66 | 20.33 | 910 |
| Example 12 | 21.5 | 0.71 | 64.88 | 3.13 | 19.73 | 870 |
| Example 13 | 21.5 | 0.71 | 64.82 | 3.15 | 19.78 | 870 |

(continued)

| Calcined body | Sintering shrinkage rate (%) | Sintered body | | | | |
| --- | --- | --- | --- | --- | --- | --- |
| | | Proportion of total light transmittance | Color tone | | | Three-point bending strength (MPa) |
| | | | L* | a* | b* | |
| Example 14 | 21.3 | 0.67 | 65.28 | 3.57 | 19.72 | 850 |

The standard deviation of the chroma C* of the five sintered bodies of each of Examples 9 to 14 is presented in a table below together with the evaluation results of Comparative Example 1.

[Table 12]

| | Standard deviation of C* |
| --- | --- |
| Example 9 | 0.04 |
| Example 10 | 0.06 |
| Example 11 | 0.04 |
| Example 12 | 0.05 |
| Example 13 | 0.05 |
| Example 14 | 0.06 |
| Comparative Example 1 | 0.12 |

The standard deviations of C* in Examples 9 to 14 were all smaller than that in Comparative Example 1 and were comparable to each other. Thus, it can be seen that the reproducibility of the color tone with respect to variations in sintering temperature is high as compared with the conventional sintered bodies, regardless of the BET specific surface area of the powder, the amount of the stabilizing element, and the presence or absence of alumina.

The entire contents of the description, claims and abstract of Japanese Patent Application No. 2022-010606 filed on January 27, 2022 are hereby incorporated by reference as the disclosure of the description of the present disclosure.

REFERENCE SYMBOLS

1    calcined body
2    sagger

**Claims**

1. A powder of zirconia, comprising a stabilizing element and a transition metal element, wherein in a frequency distribution of element ratios of the transition metal element/zirconium plotted at intervals of 0.005, a difference between a minimum value and a maximum value of the transition metal element/zirconium is less than 0.25.

2. The powder according to claim 1, wherein the transition metal element is one or more selected from the group consisting of titanium (Ti), chromium (Cr), manganese (Mn), iron (Fe), cobalt (Co), nickel (Ni), copper (Cu), niobium (Nb), vanadium (V), molybdenum (Mo), technetium (Tc), ruthenium (Ru), rhodium (Rh), palladium (Pd) and silver (Ag).

3. The powder according to claim 1 or 2, wherein the stabilizing element is one or more selected from the group consisting of yttrium (Y), calcium (Ca), magnesium (Mg), terbium (Tb) and erbium (Er).

4. The powder according to any one of claims 1 to 3, wherein the powder contains one or more selected from the group consisting of alumina ($Al_2O_3$), silica ($SiO_2$) and germania ($GeO_2$).

5. The powder according to any one of claims 1 to 4, wherein a total frequency of the transition metal element/zirconium of 0.05 or more in the frequency distribution is 2.5% or less.

6. The powder according to any one of claims 1 to 5, wherein the powder has a BET specific surface area of 8 $m^2/g$ or more and 15 $m^2/g$ or less.

7. The powder according to any one of claims 1 to 6, wherein the powder has a bulk density of 1.10 $g/cm^3$ or more and 1.40 $g/cm^3$ or less.

8. The powder according to any one of claims 1 to 7, wherein when a disk-shaped green body obtained by filling 3.0 g of the powder into a mold having a diameter of 25 mm, performing uniaxial pressing at a pressure of 49 MPa and then performing CIP treatment at a pressure of 196 MPa is calcined under the following conditions to provide a calcined body, a shrinkage percentage determined from the following formula is less than 4.0%:

   calcination temperature: 1,000°C,
   calcination time: 1 hour,
   rate of temperature increase: 50 °C/hour,
   calcination atmosphere: air atmosphere,
   rate of temperature decrease: 300 °C/hour and

   $$\text{shrinkage percentage [\%]} = \{(25 - \text{diameter of calcined body}) \text{ [mm]}/25 \text{ [mm]}\} \times 100$$

   (1).

9. The powder according to any one of claims 1 to 8, wherein the powder is a granulated powder.

10. A method for producing the powder according to any one of claims 1 to 8, comprising a step of drying a composition containing hydrated zirconia, a stabilizing element source, a transition metal element source and a solvent to provide a dry powder, and a step of subjecting the dry powder to heat treatment at a temperature lower than a sintering temperature to provide a calcined powder.

11. The method according to claim 10, wherein the transition metal element source is at least one of an oxide and a chloride of one or more selected from the group consisting of titanium (Ti), chromium (Cr), manganese (Mn), iron (Fe), cobalt (Co), nickel (Ni), copper (Cu), niobium (Nb), molybdenum (Mo), technetium (Tc), ruthenium (Ru), rhodium (Rh), palladium (Pd), and silver (Ag).

12. The method according to claim 10 or 11, wherein the heat treatment temperature in the heat treatment is 1,200°C or lower.

13. A green body, comprising the powder according to any one of claims 1 to 9.

14. A method for producing a calcined body, comprising a step of calcining the green body according to claim 13.

15. A method for producing a sintered body, comprising a step of sintering at least one of a green body containing the powder according to any one of claims 1 to 9 and a calcined body obtained by calcining the green body.

FIG. 1

FIG. 2

FIG. 3

FIG. 4

FIG. 5

FIG. 6

FIG. 7

FIG. 8

FIG. 9

FIG. 10

## INTERNATIONAL SEARCH REPORT

International application No.

**PCT/JP2023/002253**

### A. CLASSIFICATION OF SUBJECT MATTER

*C04B 35/488*(2006.01)i; *A61C 13/00*(2006.01)i; *A61C 13/08*(2006.01)i; *C01G 25/02*(2006.01)i
FI:   C04B35/488; C01G25/02; A61C13/00 A; A61C13/08 Z

According to International Patent Classification (IPC) or to both national classification and IPC

### B. FIELDS SEARCHED

Minimum documentation searched (classification system followed by classification symbols)

C04B35/488; A61C13/00; A61C13/08; C01G25/02

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 5-193948 A (TOSOH CORP.) 03 August 1993 (1993-08-03) paragraphs [0017], [0019], [0024]-[0029], example 1 | 1-4, 6, 9-15 |
| A | entire text, all drawings | 5, 7, 8 |
| X | JP 5-155622 A (TOSOH CORP.) 22 June 1993 (1993-06-22) paragraphs [0012], [0015]-[0018], example 1 | 1-4, 6, 7, 9, 10-15 |
| A | entire text, all drawings | 5, 8 |
| X | JP 2017-145158 A (TOSOH CORP.) 24 August 2017 (2017-08-24) paragraphs [0060], [0061], [0063], example 1 | 1, 3, 9, 10, 12-15 |
| A | entire text, all drawings | 2, 4, 5-8, 11 |
| A | JP 2016-060687 A (KURARAY NORITAKE DENTAL INC.) 25 April 2016 (2016-04-25) paragraphs [0024], [0041] | 1-15 |
| A | JP 2020-001973 A (KURARAY NORITAKE DENTAL INC.) 09 January 2020 (2020-01-09) entire text, all drawings | 1-15 |

☑ Further documents are listed in the continuation of Box C.     ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **03 April 2023** | **18 April 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
|---|
| **PCT/JP2023/002253** |

### C.    DOCUMENTS CONSIDERED TO BE RELEVANT

| Category* | Citation of document, with indication, where appropriate, of the relevant passages | Relevant to claim No. |
|---|---|---|
| A | JP 2016-117618 A (KURARAY NORITAKE DENTAL INC.) 30 June 2016 (2016-06-30) entire text, all drawings | 1-15 |

Form PCT/ISA/210 (second sheet) (January 2015)

**INTERNATIONAL SEARCH REPORT**
**Information on patent family members**

| International application No. |
| --- |
| **PCT/JP2023/002253** |

| Patent document cited in search report | | | Publication date (day/month/year) | Patent family member(s) | Publication date (day/month/year) |
| --- | --- | --- | --- | --- | --- |
| JP | 5-193948 | A | 03 August 1993 | (Family: none) | |
| JP | 5-155622 | A | 22 June 1993 | (Family: none) | |
| JP | 2017-145158 | A | 24 August 2017 | (Family: none) | |
| JP | 2016-060687 | A | 25 April 2016 | (Family: none) | |
| JP | 2020-001973 | A | 09 January 2020 | (Family: none) | |
| JP | 2016-117618 | A | 30 June 2016 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9428422 B
- EP 3892254 A

- JP 2022010606 A